# EUROPÄISCHE PATENTSCHRIFT

(11) **EP 2 638 156 B1**
(45) Veröffentlichungstag und Bekanntmachung des Hinweises auf die Patenterteilung: **27.01.2016**
(21) Anmeldenummer: 11779665.6
(22) Anmeldetag: 08.11.2011
(51) Int. Cl.: C12N 15/10, C12Q 1/68

(54) **VERFAHREN UND VORRICHTUNG ZUR ISOLIERUNG UND REINIGUNG VON DOPPELSTRÄNGIGEN NUKLEINSÄUREN**
METHOD AND DEVICE FOR ISOLATING AND PURIFYING DOUBLE-STRANDED NUCLEIC ACIDS
PROCÉDÉ ET DISPOSITIF POUR L'ISOLEMENT ET LA PURIFICATION D'ACIDES NUCLÉIQUES DOUBLE BRIN

(30) Priorität: 09.11.2010 EP 10014396
(43) Veröffentlichungstag der Anmeldung: 18.09.2013
(73) Patentinhaber: Qiagen GmbH, 40724 Hilden (DE)
(72) Erfinder: SINGER, Thorsten, 42699 Solingen (DE); WEDLER, Holger, 40724 Hilden (DE)
(74) Vertreter: f & e patent
(86) Internationale Anmeldenummer: PCT/EP2011/069644
(87) Internationale Veröffentlichungsnummer: WO 2012/062753

(56) Entgegenhaltungen:
- EP-A1- 1 559 783
- EP-A1- 1 911 844
- WO-A1-01/38516
- WO-A1-99/00168
- DE-A1- 4 321 904
- DE-A1-102007 005 655
- US-A1- 2004 152 076
- BALAN SINDHU ET AL: "Metal chelate affinity precipitation of RNA and purification of plasmid DNA", BIOTECHNOLOGY LETTERS, KEW, SURREY, GB, Bd. 25, Nr. 13, 1. Juli 2003 (2003-07-01), Seiten 1111-1116, XP002413172, ISSN: 0141-5492, DOI: DOI:10.1023/A:1024148316322
- MURPHY JC ET AL: "Nucleic acid separations utilizing immobilized metal affinity chromatography", BIOTECHNOLOGY, BUTTERWORTHS, LONDON, GB, Bd. 19, Nr. 3, 20. Mai 2003 (2003-05-20), Seiten 982-986, XP002994010, ISSN: 0740-7378
- IHARA T ET AL: "DNA SEPARATION USING ZR(IV)-LOADED RESIN THROUGH LIGAND EXCHANGE", 20010101, Bd. 17, Nr. SUPPL, 1. Januar 2001 (2001-01-01), Seiten IL229-IL231, XP008016581,
- TEETERS M A ET AL: "Adsorptive membrane chromatography for purification of plasmid DNA", JOURNAL OF CHROMATOGRAPHY, ELSEVIER SCIENCE PUBLISHERS B.V, NL, Bd. 989, Nr. 1, 7. März 2003 (2003-03-07), Seiten 165-173, XP004410558, ISSN: 0021-9673, DOI: DOI:10.1016/S0021-9673(03)00027-X

## Beschreibung

Die vorliegende Erfindung betrifft eine chromatographische Vorrichtung zur Isolierung und/oder Reinigung von doppelsträngigen Nukleinsäuren, bevorzugt von doppelsträngiger DNA, die Verwendung dieser Vorrichtung zur chromatographischen Isolierung und/oder Reinigung doppelsträngiger Nukleinsäuren, bevorzugt von doppelsträngiger DNA, aus einer Mischung solcher Nukleinsäuren mit einzelsträngigen Nukleinsäuren und/oder Oligonukleotiden, Mononukleotiden, Salzen und sonstigen Verunreinigungen sowie ein Verfahren zur chromatographischen Isolierung und/oder Reinigung doppelsträngiger Nukleinsäuren, bevorzugt doppelsträngiger DNA, aus einer solchen Mischung und einen Kit hierfür.

Seit der Entdeckung der Polymerase-Kettenreaktion (polymerase chain reaction, PCR) haben Nukleinsäuren in diversen Gebieten der Medizin und Molekularbiologie enorm an Bedeutung gewonnen und werden in zunehmendem Maße u. a. in der rekombinanten Gentechnik, der medizinischen Diagnostik, der Entwicklung neuer Wirkstoffe und der Forensik eingesetzt. Die PCR zählt mittlerweile zu den wichtigsten Methoden der modernen Molekularbiologie und wird in medizinischen und biologischen Laboratorien bspw. zur Erkennung von Erbkrankheiten und Virusinfektionen, in der Gerichtsmedizin zum Erstellen genetischer Fingerabdrücke und für Vaterschaftstests verwendet sowie zur Klonierung von Genen, welche durch anschließende Exprimierung zur Herstellung von Proteinen bspw. als pharmakologisch wirksame Substanzen dienen können. Ebenso wäre das Humangenomprojekt ohne die PCR nicht denkbar gewesen.

Die PCR stellt eine Methode dar, die es ermöglicht, die Erbsubstanz Desoxyribonukleinsäure (DNA) mithilfe eines Enzyms, der DNA-Polymerase, *in vitro* zu vervielfältigen (amplifizieren). Sie wird vor allem eingesetzt, um einen relativ kurzen, genau definierten Teil eines DNA-Stranges zu vervielfältigen, wobei die dabei gebildeten Produkte im nächsten Reaktionszyklus ihrerseits ebenfalls als Edukte dienen und somit eine exponentielle Vervielfältigung des Ausgangsmaterials erzielt wird. Aufgrund der exponentiellen Vervielfältigung des Ausgangsmaterials reichen selbst kleinste Probenmengen aus, um hinreichend Material für nachfolgende Untersuchungen zu erhalten, welche im Allgemeinen deutlich größere Probenmengen erfordern. Durch Automatisierung der PCR ist es heutzutage möglich, Tausende unterschiedlicher DNA-Sequenzen binnen kürzester Zeit in paralleler Weise zu vervielfältigen.

Der Reaktionsansatz der PCR enthält neben der DNA, welche den zu vervielfältigenden Abschnitt enthält (Templat), mindestens zwei unterschiedliche Arten kurzer Oligonukleotidstränge (Primer), welche den Startpunkt der DNA-Synthese auf beiden Strängen der Ausgangs-DNA festlegen, eine DNA-Polymerase und Desoxyribonukleosidtriphosphate (dNTPs), welche als monomere Bausteine der von der DNA-Polymerase synthetisierten DNA-Stränge dienen, sowie ferner Mg²⁺-Ionen als essentielle Co-Faktoren der DNA-Polymerase und eine Pufferlösung, welche den für die DNA-Polymerase geeigneten pH-Wert in dem Reaktionsmedium sicherstellt. Nach beendeter Reaktion enthält das Reaktionsgemisch neben den doppelsträngigen Desoxyribonukleinsäuren noch überschüssige Mononukleotide, insbesondere dNTPs, und Primer, sowie das Enzym, Salze, weitere Bestandteile der Pufferlösung und eventuell weitere Verunreinigungen. All diese Substanzen können in nachfolgenden Anwendungen, bspw. Sequenzierungsreaktionen stören, indem sie diese inhibieren oder Artefakte verursachen. Insbesondere im Bereich der molekularen Diagnostik ist es daher unabdingbar, das aus der PCR erhaltene Reaktionsgemisch vor der weiteren Verwendung zu reinigen, um die genannten Verunreinigungen, wie überschüssige dNTPs, Primer, Salze etc. abzutrennen.

Aus dem Stand der Technik sind diverse Verfahren zur Aufreinigung von PCR-Produkten bekannt, insbesondere zum Entfernen überschüssiger Mononukleotide und Primer und zur Entsalzung der Probe. Die als Amplifikationsprodukt erhaltene doppelsträngige DNA (double-stranded DNA, dsDNA) kann bspw. durch Zusatz von Alkoholen wie Ethanol oder Isopropanol aus dem Reaktionsgemisch ausgefällt werden. Eine solche Fällung ist aber gerade in Hochdurchsatzverfahren, bspw. der automatisierten PCR in Multiwellplatten, aufgrund von Mischungsvorgängen und bezüglich des nachfolgenden Abtrennens des Alkohols von dem Präzipitat nicht trivial. Ferner ist diese Methode hinsichtlich des maximalen Probenvolumens der PCR limitiert, falls die Fällung direkt im Reaktionsgefäß der PCR stattfinden soll, da ein gewisses Mindestvolumen an Salz und Alkohol zu dem Reaktionsgemisch hinzugegeben werden muss, um eine effiziente Fällung zu erzielen. Soll die Fällung nicht im Reaktionsgefäß stattfinden, ist andererseits ein zusätzlicher Schritt des Umfüllens der aus der PCR erhaltenen Ansätze notwendig. Im Allgemeinen ist ferner ein mehrmaliges, zeitintensives Zentrifugieren der Probe zur Abtrennung des Niederschlages notwendig, gefolgt von einer ebenfalls zeitaufwendigen Prozedur zum Waschen der gefällten DNA, anschließendem Trocknen und einer Resolubilisierung. Hierauf basierende Verfahren sind somit quasi nicht automatisierbar, und die erhaltenen Ausbeuten sind ferner stark vom Geschick des Experimentators abhängig. Im Allgemeinen erfolgt keine vollständige Entfernung der Mononukleotide und Primer, und auch der Restsalzgehalt in der gereinigten Probe ist stark von der Anzahl und Qualität der Waschschritte abhängig.

Zur Aufbereitung der aus der PCR erhaltenen Proben für nachfolgende Sequenzierungsreaktionen kann ferner auch die sogenannte ExoSAP-Methode verwendet werden. Hierzu wird das aus der PCR erhaltene Reaktionsgemisch mit einer Mischung zweier hydrolytischer Enzyme, nämlich einer Exonuklease (I) und einer alkalischen Phosphatase, bspw. Shrimp Alkaline Phosphatase, in einem geeigneten Puffer vermischt. Während Exonuklease (I) einzelsträngige Primer und einzelsträngige DNA (single-stranded DNA, ssDNA) abbaut, dephosphoryliert die alkalische Phosphatase die verbleibenden dNTPs. In einem nachfolgenden Schritt muss die Probe 15 Minuten bei 80 °C behandelt werden, um die hydrolytischen Enzyme zu denaturieren. Während diese Methode zwar zur Aufbereitung von aus der PCR erhaltenen Reaktionsgemischen für die nachfolgende Sequenzierung geeignet ist, sind die auf diese Weise erhaltenen Proben für eine Vielzahl weiterer Reaktionen nicht geeignet, da zum einen in der Probe enthaltene Salze nicht entfernt werden und die dephosphorylierten Nukleoside, anorganischen Phosphate und deaktivierten Enzyme in der Probe verbleiben.

Ein weiteres Verfahren zur Abtrennung und Konzentrierung hochmolekularer Substanzen von niedermolekularen ist die Ultrafiltration, bei der eine Lösung, welche ein Gemisch aus niedermolekularen Substanzen (im Falle der PCR bspw. Salze, dNTPs, Lösungsmittel und Primer) und hochmolekularen Substanzen (bspw. langkettige DNA) mittels Vakuum oder Überdruck gegen eine Ultrafiltrationsmembran aus Edelstahl, Kunststoff oder textilem Gewebe mit Poren einer definierten Ausschlussgrenze (cut-off) gepresst wird. Diejenigen Substanzen, welche größer als die Membranporen sind, werden hierbei von der Membran zurückgehalten, während Substanzen, welche kleiner sind als die Membranporen, die Membran passieren können. Ein Nachteil dieser Methode ist, dass in einem anschließenden Schritt das auf der Membran verbleibende Probenretendat wieder gelöst oder suspendiert werden muss und durch Pipetieren in geeignete Gefäße zur Lagerung oder weiteren Umsetzung überführt werden muss. Ferner ist die Ultrafiltration ein rein physikalisches Trennverfahren, welches auf der Differenz der Molekülgröße der zu isolierenden Substanz und der Verunreinigungen beruht. Es lässt sich daher bspw. nicht zur Abtrennung langkettiger einzelsträngiger Nukleinsäuren, bspw. RNA, von doppelsträngigen Nukleinsäuren vergleichbarer Größe einsetzen.

Andere Verfahren basieren auf der selektiven Bindung der zu isolierenden DNA an einer stationären Phase, entweder durch Adsorption, Absorption oder kovalente chemische Bindung. In einem ersten Schritt wird die zu isolierende DNA unter geeigneten Bedingungen selektiv an die stationäre Phase gebunden. Die Verunreinigungen interagieren nicht in demselben Maße wie die zu isolierende DNA mit der stationären Phase und können daher durch geeignete Waschschritte entfernt werden. Nach dem Entfernen der Verunreinigungen ist ein weiterer Schritt zum Ablösen der DNA von der stationären Phase nötig (Desorption), in welchem durch Zugabe geeigneter Lösungen die Wechselwirkung zwischen der DNA und der stationären Phase minimiert wird. Dieses "bind-wash-elute"-Prinzip machen sich bspw. Verfahren zunutze, bei denen die zu isolierende DNA unter chaotropen Bedingungen und/oder in Gegenwart von Alkoholen an Silikapartikel gebunden wird. Hierbei muss oft ein Vielfaches des Volumens der ursprünglichen Probenlösung an chaotroper Salzlösung und/oder Alkohol zu der Probe hinzugegeben werden, bevor die DNA selektiv an die Silikapartikel gebunden werden kann. Zur Entfernung der Verunreinigungen sind mehrere Waschschritte notwendig. Um die DNA anschließend vollständig wieder von den Silikapartikeln abzulösen, ist die Zugabe eines geeigneten Niedrigsalz-Puffers notwendig. Das hierfür benötigte Volumen des Elutionspuffers führt häufig zu einer geringen Konzentration der DNA in der nach der Reinigung erhaltenen Probe, so dass zusätzliche Schritte zur Aufkonzentrierung notwendig sind. Teilweise lässt sich die gebundene DNA auch nicht mit angemessenen Volumina der Elutionslösung vollständig von den Partikeln entfernen, und es kommt zu Ausbeuteverlusten. Gerade im Bereich der Hochdurchsatzanalyse, beim Verwenden von Filterplatten, kommt es darüber hinaus häufig zu einem Einschleppen von Ethanol aus dem verwendeten Waschpuffer in die gereinigte Proben, dessen Entfernung einen zusätzlichen Arbeitsschritt zum Verdampfen des Ethanols notwendig macht.

Ein weiteres Beispiel für ein Verfahren, welches auf der sog. "bind-wash-elute"-Routine basiert, ist die Anionen-Austausch-Chromatographie (anion exchange chromatography, AEX). Anionen-Austausch-Verfahren basieren auf der Wechselwirkung zwischen den negativ geladenen Phosphaten der Nukleinsäure und der positiv geladenen Oberfläche des Anionenaustauschmaterials (Forcic et al. J. Chromatogr. A 2005, 1065 (1), 115-120). Unter Niedrigsalzbedingungen bindet die in einer Probe enthaltene DNA selektiv an die stationäre Phase, während Verunreinigungen in nachfolgenden Waschschritten unter Verwendung von Waschpuffern einer mittleren Salzkonzentration entfernt werden können. In einem weiteren Schritt kann die DNA von der stationären Phase mit einem stark salzhaltigem Puffer desorptiv eluiert werden. Aufgrund des hohen Salzgehaltes des Elutionspuffers ist anschließend eine Fällung der DNA aus dem Eluat mit Alkohol notwendig.

Verfahren, welche auf der selektiven Bindung von DNA basieren, liefern zwar im Allgemeinen DNA einer hohen Reinheit, allerdings sind hierbei stets mehrere Arbeitsschritte für das Binden, Waschen und Eluieren der DNA notwendig, was mitunter zeit- und kostenintensiv sein kann.

Ferner gab es im Stand der Technik bereits Versuche, zumindest einen Teil der jeweiligen Nachteile der einzelnen Methoden dadurch zu überwinden, dass mindestens zwei unterschiedliche der oben genannten Verfahren kombiniert wurden.

Zum einen besteht natürlich die Möglichkeit, zwei oder mehrere der oben genannten Verfahren nacheinander in getrennten Vorrichtungen auszuführen. Aus Zeit- und Kostengründen ist diese Vorgehensweise jedoch nur in solchen Fällen praktikabel, in welchen lediglich einzelne spezielle Proben hochrein erhalten werden sollen, keinesfalls jedoch in der Hochdurchsatzanalytik. Daher gab es auch vereinzelte Versuche, zwei oder mehrere der oben genannten Verfahren in einer Vorrichtung zu kombinieren.

So ist bspw. in WO 99/00168 eine Vorrichtung zur Reinigung biologischer Moleküle beschrieben, in welcher ein adsorptives Chromatographie-Medium mit einem Größenausschluss-Chromatographie-Medium kombiniert wird. Bei dem adsorptiven Medium handelt es sich bspw. um ein lonenaustausch-Chromatographie-Harz. In einem ersten Schritt wird das zu isolierende und/oder zu reinigende biologische Molekül unter den oben beschriebenen Bedingungen an dieses Medium gebunden. Durch einen oder mehrere Waschschritte werden dann die Verunreinigungen, die nicht in dem gleichen Maße wie das zu isolierende Molekül mit dem Chromatographie-Medium wechselwirken, durch Auswaschen von diesem getrennt. Zuletzt wird durch einen geeigneten Elutionspuffer das gewünschte Molekül von dem adsorptiven Medium desorbiert. Gegenüber den oben genannten Verfahren zeichnet sich das in WO 99/00168 beschriebene dadurch aus, dass das adsorptive Medium mit einem nachfolgenden Größenausschluss-Chromatographie-Medium kombiniert wird. Hierdurch können unter geeigneten Bedingungen niedrigmolekulare Komponenten aus der mobilen Phase entfernt werden. Sofern das Volumen des desorptiven Elutionspuffers genau kontrolliert wird, kann das zu isolierende Molekül in einem entsalzten Eluat erhalten werden und ein zusätzlicher Schritt zum Ausfällen des biologischen Moleküls entfällt, welcher ein Nachteil konventioneller lonen-Austausch-Chromatographie ist. Nichtsdestotrotz ist auch diese Methode noch vergleichsweise zeitaufwendig, da mindestens drei Schritte zum Binden, Waschen und desorptiven Eluieren des gewünschten Moleküls durchgeführt werden müssen. Darüber hinaus muss das zu isolierende Molekül deutlich größer als die Verunreinigungen sein, damit es das Größenausschluss-Chromatographie-Medium im desorptiven Elutionsschritt schnell genug passieren kann, ohne dass kleinere Moleküle, welche bereits während des Anbringens des Zielmoleküls an die adsorptive Phase oder des Waschens in die Poren des Größenausschluss-Chromatographie-Mediums eingedrungen sind, nicht im desorptiven Elutionsschritt gemeinsam mit dem Zielmolekül eluiert werden.

Weiterhin sind in WO 2004/011142 sogenannte SEIE-Partikel beschrieben, welche ebenfalls die Vorteile der Größenaustauschchromatographie mit den Vorteilen der lonen-Austausch-Chromatographie kombinieren sollen. Hierbei handelt es sich bevorzugt um Partikel, bei denen ein Kern aus einem lonen-Austausch-Chromatographie-Medium (IEX-Medium) in eine Hülle aus einem Größenausschluss-Chromatographie-Medium (SEC-Medium) eingeschlossen ist. Kleine Moleküle wie anorganische Ionen und dNTPs können durch die Großenausschlusshülle hindurchdringen und werden in dem Kern der Partikel durch das lonenaustausch-Medium zurückgehalten, während größere Moleküle, wie lange einzelsträngige Nukleinsäuren oder doppelsträngige Nukleinsäuren zu groß sind, um die Poren des Größenaustausch-Mediums zu penetrieren und daher in Lösung verbleiben. Die in WO 2004/011142 genannten Vorrichtungen eignen sich zwar zur Entfernung von Mononukleotiden (dNTPs), kurzen Oligonukleotidprimern, Salzen und Metallionen aus einer biologischen Probe, jedoch können mit dieser Methode keine Verunreinigungen abgetrennt werden, die in ihrer Größe bzw. ihrem hydrodynamischem Radius den zu isolierenden doppelsträngigen Nukleinsäuren vergleichbar sind. Letztendlich beruht Trennung hauptsächlich auf der Molekülgröße.

Alle aus dem Stand der Technik bekannten Verfahren zur Aufreinigung von DNA, insbesondere zur Reinigung von PCR-Produkten, umfassen in der Regel somit mehrere Arbeitsschritte, was allein diese Methoden zeitaufwändig macht. Darüber hinaus sind die bekannten Verfahren zum Teil sehr arbeits- und kostenintensiv und fehleranfällig. Insbesondere für Hochdurchsatzverfahren werden darüber hinaus zur Automation spezielle Geräte benötigt, deren Herstellungs- und Wartungskosten mit der Anzahl der von ihnen durchzuführenden unterschiedlichen Schritte stark ansteigt. Keine der bisher bekannten Schritte erfüllt daher alle Anforderungen an eine schnelle und reproduzierbare Reinigung von doppelsträngigen Nukleinsäuren, insbesondere von PCR-Produkten, welche Produkte hoher Qualität bei guter Reproduzierbarkeit liefert.

Die Aufgabe der vorliegenden Erfindung war es daher, eine Vorrichtung und ein Verfahren zur Verfügung zu stellen, welches die Isolierung und/oder Aufreinigung von doppelsträngigen Nukleinsäuren, insbesondere von als Amplifikationsprodukten einer PCR erhaltenen doppelsträngigen DNA in einem einzigen Arbeitsschritt ermöglicht und dabei sowohl einzelsträngige Nukleinsäuren, bspw. als Primer verwendete Oligonukleotide, Mononukleotide, insbesondere dNTPs, und Salze in einem einzigen Reaktionsschritt zuverlässig entfernt, ohne dass nachfolgende Schritte zur Resolubilisierung oder Resuspension der DNA, zum Transfer der gereinigten Probe oder zum Aufkonzentrieren nötig wären.

Es ist nun überraschenderweise gefunden worden, dass dies mithilfe einer chromatographischen Vorrichtung zur Isolierung und/oder Reinigung von doppelsträngigen Nukleinsäuren, bevorzugt von doppelsträngiger DNA erzielt werden kann, umfassend: (1) einen Grundkörper, (2) mindestens einen Hohlraum innerhalb des Grundkörpers, welcher mit einem Einlass und einem Auslass versehen ist und (3) eine stationäre Phase, welche sich in dem Hohlraum befindet, wobei die stationäre Phase wenigstens mindestens ein poröses Chromatographie-Harz, welches als Größenausschluss-Chromatographie-Medium fungiert, und immobilisierte Metallionen umfasst.

Gegenüber den aus dem Stand der Technik bekannten Vorrichtungen und Verfahren zur Isolierung und/oder Reinigung doppelsträngiger DNA, bspw. zur Isolierung der Amplifikationsprodukte einer PCR aus dem Reaktionsgemisch, zeichnen sich die erfindungsgemäße Vorrichtung und das erfindungsgemäße Verfahren durch eine Reihe von Vorteilen aus, wie die nachfolgende Tabelle 1 verdeutlicht.

**Tabelle 1**

| | Alkoholfällung | ExoSAP | MinElute spin | MinElute vacuum | QIAquick spin | QIAquick vacuum | QIAquick 96 vacuum | MinElute 96 UF | vorliegende Erfindung |
|---|---|---|---|---|---|---|---|---|---|
| *Anzahl der erforderlichen Schritte* | | | | | | | | | |
| Plpettieren | 2 bis 3 | 3 bis 4 | 4 | 4 | 4 | 4 | 4 | 3 | 0 bis 1 |
| Mischen | 1 | 2 | 1 | 1 | 1 | 1 | 1 | 1 | 0 |
| Unterdruck/Vakuum | 1 | 1 | 0 | 3 | 0 | 3 | 3 | 1 | 0 |
| Zentrifugieren | 2 | 2 | 4¹ | 1 | 4¹ | 1 | 1 | 0 | 21 |
| Summe der Schritte | 6 bis 7 | 8 bis 9 | 9¹ | 9 | 9¹ | 9 | 9 | 5 | 2 bis 3¹ |
| Inkubation (min) | 0 | 2x15 | 1 | 1 | 1 | 1 | 1 | 0 | 1 |
| Zahl der Puffer / sonstigen Lösungen pro Kit | 2 bis 3 | 3 bis 4 | 3 | 3 | 3 | 3 | 3 | 2 | 1 |
| benötigte Zeit (min) | 20 bis 45 | ca. 30 | 5 bis 10 | 5 bis 15 | 5 bis 10 | 5 bis 15 | 25 | 20 | 5 bis 10 |
| Qualität der erhaltenen | gering bis mittel | mittel bis gut | sehr gut | gut bis sehr gut | sehr gut | gut bis sehr gut | gut bis sehr gut | gut bis sehr gut | sehr gut |
| Ausbeute | <=100% | <=100% | >80% | >80% | >80% | >80% | >80% | >90% | 100% |
| hauptsächliche Nachteile | langes Zentrifugieren | langes Zentrifugieren | geringer Durchsatz | Übertrag von Ethanol und Salz in die gereinigte Probe | geringer Durchsatz | Übertrag von Ethanol und Salz in die gereinigte Probe | Übertrag von Ethanol und Salz in die gereinigte Probe | abschließen-des Pipettieren | |

| | | | | | | | | | |
|---|---|---|---|---|---|---|---|---|---|
| ¹ Das sog. "Prespinning" zum Entfernen überschüssigen Lösungsmittels aus der stationären Phase vor dem Auftragen der Probe auf die stationäre Phase bereits eingerechnet | | | | | | | | | |

Die Systeme ExoSAP (Fa, USB Corporation, Cleveland, Ohio, USA) MinElute spin, vacuum und 96 UF (Qiagen, Hilden, Deutschland) sowie QIAquick spin, vacuum und 96 vaccum (Qiagen, Hilden, Deutschland) sind kommerziell erhältlich, und Protokolle zur ihrer Verwendung sind vom Hersteller erhältlich. Standardprotokolle für die Alkoholfällung sind dem Fachmann bekannt. Gegenüber diesen Vorrichtungen bzw. Verfahren, die auf ihrer Verwendung beruhen, zeichnen sich das erfindungsgemäße System und das erfindungsgemäße Verfahren dadurch aus, dass dsDNA in sehr kurzer Zeit (5 bis 10 Minuten) in quantitativer Ausbeute und sehr guter Qualität in nur zwei bis drei Arbeitsschritten erhalten werden kann, wobei der vorbereitende Schritt des sog. "Prespinnings" bei Verwendung einer sog. Zentrifugationssäule (spin column) zum Entfernen überschüssigen Lösungsmittels aus der stationären Phase vor dem Auftragen der Probe auf dieselbe bereits eingerechnet ist. Als zusätzliche Lösung wird lediglich eine Pufferlösung zum Einstellen des pH-Wertes benötigt.

Die erfindungsgemäße Vorrichtung ermöglicht die chromatographische Isolierung und/oder Reinigung von doppelsträngigen Nukleinsäuren durch Entfernung von Verunreinigungen und weiteren Probenbestandteilen wie einzelsträngigen Nukleinsäuren, Mononukleotiden, insbesondere dNTPs, Nukleosiden und Salzen in einem einzigen chromatographischen Schritt, indem sie zwei Reinigungsprinzipien in einer stationären Phase vereint.

Im Sinne der Erfindung werden unter doppelsträngigen Nukleinsäuren doppelsträngige DNA wie auch doppelsträngige Hybride aus DNA und RNA und doppelsträngige RNA jedweder Kettenlänge verstanden, insbesondere auch Plasmid-DNA (pDNA), genomische DNA (gDNA) oder Fragmente davon. Bevorzugt handelt es sich bei den doppelsträngigen Nukleinsäuren um doppelsträngige DNA, welche als Amplifikationsprodukt einer PCR erhalten wurde.

Im Sinne der Erfindung wird als stationäre Phase die Gesamtheit der innerhalb der chromatographischen Vorrichtung enthaltenen Chromatographie-Medien verstanden, umfassend ein oder mehrere Chromatographie-Medien und immobilisierte Metallionen, unabhängig davon, ob es sich hierbei um ein einziges Chromatographie-Medium, eine homogene Mischung mehrerer Chromatographie-Medien oder einer Anordnung mehrerer Chromatographie-Medien in mehreren Schichten handelt.

Einzelsträngige Nukleinsäuren, bspw. einzelsträngige DNA in Form überschüssiger Amplifikationsprimer einer PCR, werden durch Wechselwirkung der immobilisierten Metallionen mit den aromatischen Stickstoffbasen des Einzelstranges selektiv an die stationäre Phase gebunden und so aus der mobilen Phase entfernt, welche die stationäre Phase während der chromatographischen Reinigung passiert. Auch Mononukleotide, insbesondere überschüssige dNTPs, können durch Wechselwirkungen mit immobilisierten Metallionen aus der mobilen Phase entfernt werden. Während einzelsträngige Nukleinsäuren wie Oligonukleotide, lange ssDNA und RNA sowie Mononukleotide eine starke Wechselwirkung mit den immobilisierten Metallionen zeigen, ist diese Wechselwirkung in doppelsträngigen Nukleinsäuren gering, da ein solcher Doppelstrang durch Wasserstoffbrückenbindungen der jeweils komplementärer Basen der beiden einzelnen Stränge gebildet wird, die Basen folglich im Inneren des Doppelstranges liegen und somit nach Außen abgeschirmt sind. Doppelsträngige Nukleinsäuren werden daher nicht an die stationäre Phase gebunden, sondern verbleiben in der mobilen Phase und passieren mit dieser die chromatographische Vorrichtung.

Neben immobilisierten Metallionen umfasst die stationäre Phase der erfindungsgemäßen chromatographischen Vorrichtung auch ein poröses Chromatographie-Harz, welches als Größenausschluss-Chromatographie-Medium fungiert. Die Größen-ausschlusschromatographie (size exclusion chromatography, SEC) ist eine chromatographische Methode, welche die Trennung unterschiedlicher Moleküle anhand ihrer Größe bzw. ihres hydrodynamischen Volumens.ermöglicht. Wird ein organisches Lösungsmittel als mobile Phase verwendet, bezeichnet man die Größenausschlusschromatographie auch als Gelpermeationschromatographie (GPC), bei Verwendung einer wässrigen mobilen Phase (Wasser, wässriges organisches Lösungsmittel oder ein wässriger Puffer) als Gelfiltrationschromatographie. Als stationäre Phase wird bei der Größenausschlusschromatographie eine Matrix verwendet, welche durch Quellen in einem geeigneten Lösungsmittel ein Gelbett bildet. Als Matrix werden häufig quervernetzte Silikate.oder quervernetzte organische Polymere verwendet, welche beim Quellen in einem geeigneten Lösungsmittel aufgrund der Quervernetzung in ihrem Inneren Poren definierter Größe bilden.

Während kleine Moleküle in diese Poren eindringen können und das Gelbett langsamer als die mobile Phase passieren, können große Moleküle dies nicht und passieren die stationäre Phase mit dem Lösungsmittel der mobilen Phase, also deutlich schneller als diejenigen Moleküle, welche im Inneren der Poren zurückgehalten werden. Die Größenausschlussgrenze (size-exclusion limit) eines Größenausschluss-Chromatographie-Mediums gibt das Molekulargewicht an, oberhalb welchem die in der mobilen Phase enthaltenen Moleküle zu groß sind, um in die stationären Phase einzudringen. Diese Größenausschlussgrenze wird durch die Größe der Poren innerhalb der Gelmatrix bestimmt und kann durch den Grad der Quervemetzung des Gels eingestellt werden. Heutzutage sind eine Vielzahl stationärer Phasen für die Größenausschlusschromatographie mit unterschiedlichem Grad an Quervemetzung (Größenausschlussgrenzen) kommerziell erhältlich.

Durch die Kombination eines Größenausschluss-Chromatographie-Mediums mit immobilisierten Metallionen werden ferner Metallionen, deren Ablösung von der stationären Phase während der traditionellen immobilisierten Metallionen-Affinitätschromatographie (IMAC-Chromatographie) zum Teil beobachtet wird (sog. "Ausbluten"), unmittelbar aus der mobilen Phase entfernt und gelangen nicht ins Eluat, wo sie nachfolgende Anwendungen stören könnten, da bspw. zweiwertige Metallkationen als Inhibitoren in nachfolgenden Assays zum Nachweis der isolierten Nukleinsäuren wirken können.

Um die stationäre Phase innerhalb der chromatographischen Vorrichtung zurückzuhalten, umfasst die chromatographische Vorrichtung bevorzugt mindestens einen porösen Filter, eine poröse Fritte oder eine Membran, der/die zwischen dem Auslass des Hohlraums und der stationären Phase angeordnet ist und die stationäre Phase in dem Hohlraum zurückhält. Die Porengröße dieses Filters, dieser Fritte oder dieser Membran ist hierbei größer als die zu isolierenden und/oder reinigenden doppelsträngigen Nukleinsäuren, so dass diese mit der mobilen Phase nach erfolgter Elution aus dem Auslass austreten und nicht wie bei der Ultrafiltration von der Membran zurückgehalten werden.

Bevorzugt umfasst die chromatische Vorrichtung mindestens eine Verschlussvorrichtung zum Verschließen des Einlasses und/oder des Auslasses des Hohlraums. Wenn sowohl der Einlass als auch der Auslass mit einer solchen Verschlussvorrichtung ausgestattet sind, können die Verschlussvorrichtung, welche zum Verschließen des Einlasses verwendet wird und diejenige, welche zum Verschließen des Auslasses verwendet wird, gleich oder verschieden sein.

In einer bevorzugten Ausführungsform ist die Verschlussvorrichtung eine entfernbare Einweg-Verschlussvorrichtung, ausgewählt aus der Gruppe bestehend aus abziehbaren Folien, Abrissdeckeln oder Verschlussenden, welche durch Verdrehen entlang einer Sollbruchstelle von der chromatographischen Vorrichtung abgebrochen werden können, ohne hierauf beschränkt zu sein. Die mindestens eine Verschlussvorrichtung kann ferner als wiederverwendbare Verschlussvorrichtung ausgebildet sein, bspw. in Form von Dreh- bzw. Schraubverschlüssen, Schnappdeckeln oder Stopfen, ohne auf diese beschränkt zu sein.

In einer bevorzugten Ausführungsform sind sowohl der Einlass als auch der Auslass der chromatographischen Vorrichtung mit jeweils einer Verschlussvorrichtung versehen und der von dem Grundkörper gebildete Hohlraum enthält die stationäre Phase, welche in einem Lösungsmittel vorgequollen ist, ausgewählt aus der Gruppe bestehend aus Wasser, homogenen Mischungen von organischen Lösungsmitteln mit Wasser und wässrigen Pufferlösungen. Bei dieser bevorzugten Ausführungsform wird das überschüssige Lösungsmittel bevorzugt unmittelbar vor der Verwendung der chromatographischen Vorrichtung zur Isolierung und/oder Reinigung von doppelsträngigen Nukleinsäuren entfernt.

Die chromatographische Vorrichtung ist nicht auf eine spezielle Form beschränkt, sondern jede Ausgestaltung, die üblicherweise für chromatographische Vorrichtungen Anwendung findet, kann verwendet werden, solange sie mindestens einen Hohlraum innerhalb des Grundkörpers umfasst, welcher mit einem Einlass und einem Auslass versehen ist, in welchen eine stationäre Phase eingefüllt werden kann. Beispielsweise kann die chromatographische Vorrichtung ausgewählt sein aus Glas- oder Kunststoffsäulen, welche üblicherweise für die Über- und Unterdruckchromatographie verwendet werden, Zentrifugationssäulen (sog. spin columns) oder auch Multiwellplatten, ohne darauf beschränkt zu sein. Im Allgemeinen werden chromatographische Säulen verwendet, die einen kreisförmigen Querschnitt aufweisen und deren Durchmesser im Verhältnis zu ihrer Länge gering ist Derartige Säulen können bspw. eine zylindrische oder konische Form aufweisen.

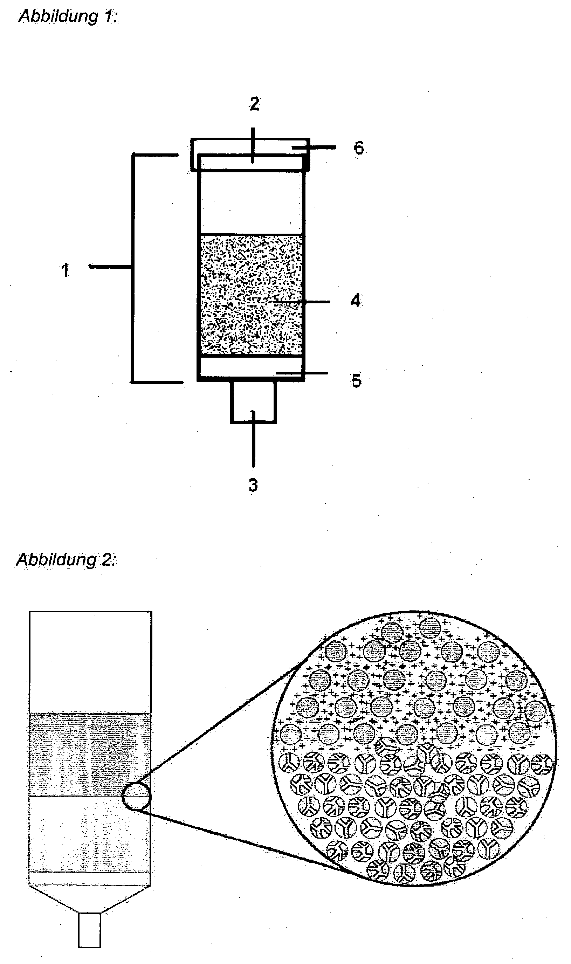

Eine bevorzugte Ausführungsform einer geeigneten chromatographischen Vorrichtung ist in Abbildung 1 gezeigt die chromatographische Vorrichtung umfasst einen Grundkörper (1), welcher in seinem Inneren einen Hohlraum definiert, der mit einem Einlass (2) und einem Auslass (3) versehen ist und in dem sich die stationäre Phase (4) befindet, sowie eine poröse Fritte (5), welche die stationäre Phase (4) in dem Hohlraum zurückhält. Der Einlass (2) der chromatographischen Vorrichtung ist mit einer Verschlussvorrichtung (6) in Form eines Deckels versehen.

Die erfindungsgemäße chromatographische Vorrichtung kann weiterhin mindestens ein Auffanggefäß zum Auffangen der nach erfolgter Elution aus dem Auslass austretenden mobilen Phase (Eluat) umfassen. Die Form und Größe des Auffanggefäßes richtet sich zweckmäßigerweise nach der Menge der aus der chromatographischen Vorrichtung austretenden mobilen Phase und der Form und Größe der chromatographischen Vorrichtung. Dem Fachmann sind eine Vielzahl geeigneter Auffanggefäße aus dem Stand der Technik bekannt. Umfasst die chromatographische Vorrichtung bspw. genau einen mit einer stationären Phase gefüllten Hohlraum, bspw. in Form einer einzigen Zentrifugationssäule, so ist zweckmäßigerweise auch nur ein Auffanggefäß zum Auffangen des Eluates umfasst. Ist die chromatographische Vorrichtung hingegen in Form mehrerer parallel angeordneter Hohlräume ausgebildet, die jeweils eine stationäre Phase enthalten, bspw. in Form einer Multiwellplatte, so sind vorteilhafterweise auch mehrere Auffanggefäße umfasst, bevorzugt in Form einer Multiwellplatte. Darüber hinaus können auch noch zusätzliche Auffanggefäße enthalten sein, bspw. zum Auffangen des überschüssigen Lösungsmittels beim Packen der Säule.

In einer bevorzugten Ausführungsform sind die immobilisierten Metallionen zwei-, drei- und oder vierwertige Metallkationen oder Mischungen davon, bevorzugt ausgewählt aus der Gruppe bestehend aus Zn²⁺, Co²⁺, Cu²⁺, Ni²⁺, Fe²⁺, Mn²⁺, Al³⁺ Co³⁺, Ga³⁺ und Zr⁴⁺ oder eine Kombination derselben. Besonders bevorzugt umfassen die immobilisierten Metallionen wenigstens ein zweiwertiges Metallkation, ausgewählt aus der Gruppe, bestehend aus Zn²⁺, Co²⁺, Cu²⁺, Ni²⁺, Fe²⁺ und Mn²⁺, optional in Kombination mit Al³⁺-Ionen, und insbesondere wenigstens Cu²⁺-Ionen, optional in Kombination mit Al³⁺-Ionen. Allerdings ist auch eine Ausführungsform mit nur zweiwertigen Metallkationen für die Erfindung geeignet, also ohne die Anwesenheit von drei- oder vierwertigen Ionen. Auch hierbei sind die oben genannten zweiwertigen Ionen bevorzugt, entweder einzeln oder in Kombination aus zweien oder mehreren der oben genannten.

Bevorzugt sind die Metallionen mithilfe wenigstens eines Linkers an wenigstens einen Teil der stationären Phase immobilisiert. Hierzu eigenen sich insbesondere Linker der allgemeinen Formel A-R-Z, in der A eine Ankergruppe zur kovalenten Anbindung des Linkers an wenigstens einen Teil der stationären Phase darstellt, Z eine mehrzähnige Kopfgruppe zur Chelatisierung der Metallionen und R eine lineare, verzweigte oder zyklische Kohlenwasserstoffkette darstellt, welche die Ankergruppe A und die Kopfgruppe Z verbindet und die substituiert oder unsubstituiert sein kann und in der eine oder mehrere Kohlenstoffatome durch Heteroatome wie O, N, S, Se ausgetauscht sein können.

Geeignete Ankergruppen zur kovalenten Anbindung des Linkers an die stationäre Phase umfassend bspw. -OH,-CO₂H, -NH₂, deren Derivate wie Säurechloride, Ester, insbesondere Aktivester, Anhydride etc., und weitere

Gruppen, sofern sie unter Ausbildung einer kovalenten Bindung mit der Matrix der stationären Phase, bspw. Sepharose oder Polyacrylamid, umgesetzt werden können.

Die Gruppe R dient vor allem als Abstandshalter zwischen der Ankergruppe und der chelatisierenden Kopfgruppe und unterliegt daher weder in ihrer Länge noch hinsichtlich ihrer Struktur besonderen Anforderungen, solange sie unter den Bedingungen, welche zur kovalenten Anbindung des Linkers an die stationäre Phase verwendet werden, der nachfolgenden Beladung des Linkers mit den Metallionen und der chromatographischen Isolierung und Reinigung der doppelsträngigen Nukleinsäuren an dieser Phase stabil ist. R kann bspw. eine Alkandiylgruppe, eine Alkendiylgruppe, eine Alkindiylgruppe, eine Aryldiylgruppe, eine Alkaryldiylgruppe oder eine Aralkandiylgruppe darstellen. In diesen können weiterhin ein öder mehrere Kohlenstoffatome durch Heteroatome ausgetauscht sein, und R kann bspw. auch ein Abstandshalter auf PEG- oder PPG-Basis sein (-(OCH₂CH₂)ₙO- bzw. - (OCH₂CH₂CH₂)ₙO-, wobei n eine positive, ganze Zahl ist).

Die Kopfgruppe Z fungiert als mehrzähniger Ligand zur Komplexierung der Metallionen, das heißt in der Kopfgruppe sind mindestens zwei funktionelle Gruppen mit freien Elektronenpaaren vorhanden, welche mindestens zwei Koordinationsstellen des Metallions unter Ausbildung koordinativer Bindungen besetzen können. Gegenüber Komplexen mit einzähnigen, untereinander nicht verknüpften Liganden zeichnen sich Chelatkomplexe aufgrund der geringeren Entropieabnahme bei der Komplexbildung und der erhöhten Wahrscheinlichkeit der Rekombination nach Ablösung eines Elektronenpaares aus der Koordinationssphäre des Zentralatoms durch eine höhere Stabilität aus. Geeignete Kopfgruppen sind daher solche Gruppen, welche mindestens zwei funktionelle Gruppen mit freien Elektronenpaaren aufweisen. Diese funktionellen Gruppen können gleich oder unterschiedlich sein und sind bevorzugt ausgewählt aus der Gruppe enthaltend -CO₂H, -CO₂R¹, -CO₂NH₂, -CONHR¹, -CONR²R³, -CR¹R²R³NR¹R², -SO₃H, -PO₃H₂, -SH, -OH, -NH₂ und -OR¹, wobei R¹, R² und R³ jeweils einen geeigneten organischen Rest darstellen, wobei bevorzugt jedes R¹, R² und R³ unabhängig voneinander ausgewählt sein kann aus linearen, (wenn es die Anzahl der Kohlenstoffe zulässt) cyclischen, gesättigten oder ungesättigten und ggf. substituierten C₁- C₂-, C₃-, C₄-, C₅-, C₆-, C₇-, C₈-, C₉- oder C₁₀-Kohlenwasserstoffketten.

In einer bevorzugten Ausführungsform ist der Linker ausgewählt aus der Gruppe bestehend aus Iminodiessigsäure (IDA), Nitrilotriessigsäure (NTA), *N*-carboxymethylierte Asparaginsäure (CM-Asp) und Tris-(2-ethylaminoethyl)-amin (TREN).

Die Verwendung immobilisierter Metallionen zur chromatographischen Reinigung von Proteinen mit Histidinmotiven, insbesondere von Proteinen mit Hexahistidin-Sequenzen (6xHis-Tag) ist seit den Siebziger Jahren bekannt und mittlerweile eine weit verbreitete Methode zur Reinigung rekombinanter Proteine, welche auf der selektiven Wechselwirkung der Stickstoffatome der heteroaromatischen Imidazolseitenkette des Histidins mit immobilisierten Metallionen wie Ni²⁺ oder Cu²⁺ beruht. Die potentielle Verwendung der immobilisierten Metallaffinitäts-Chromatographie zur Reinigung von Nukleinsäuren, deren Basen ebenfalls heteroaromatische Stickstoffatome aufweisen, wurde hingegen lange Zeit nicht untersucht. Erste Versuche zur Reinigung von DNA-Molekülen, welche mit einer speziellen Erkennungssequenz (sog. Affinitätslabel) aus sechs sukzessiven 6-Histaminylpurinresten, vergleichbar dem 6xHis-Tag der Proteine, modifiziert worden waren, beschrieben Min *et al*. (C. Min et al. Nucleic Acids Research 1996, 24 (19), 3806-3810). Der Nachteil dieses Verfahrens ist die Verwendung eines Affinitätslabels, welches zusätzliche Schritte zur Einführung und Entfernung desselben nötig macht.

Ferner ist die Verwendung eines Zr(IV)-beladenen Phosphonat-Harzes als Adsorptionsmedium für die chromatographische Reinigung von DNA in einer bind-wash-elute-Routine beschrieben worden (T. Ihara et al. Analytical Sciences 2001, 17 (Suppl.), i1229-i1231). Die generellen Nachteile sog. bind-wash-elute-Verfahren, vor allem die zusätzliche Anzahl an Verfahrensschritten, sind bereits dargelegt worden.

In der Literatur beschrieben sind darüber hinaus Verfahren zur selektiven Ausfällung von RNA in Gegenwart linearer DNA und Plasmid-DNA mithilfe von Kupfer-beladenen Polymeren in Salzlösung (S. Balan et al. Biotechnology Letters 2003, 25, 1111-1116). Dieses Verfahren erfordert jedoch zusätzliche Schritte zum Abtrennen des Niederschlags und zum Entsalzen der in dem Überstand verbleibenden DNA.

Auch die Abtrennung von RNA aus Lysaten, welche durch die Lyse von *E. coli*-Bakterien erhalten wurden, ist beschrieben worden. (J. C. Murphy et al. Biotechnol. Prog. 2003, 19, 982-986). Die Autoren beschrieben ferner, dass die Abtrennung von Oligonukleotid-Primern und PCR-Produkten mit Basenfehlpaarungen aus PCR-Ansätzen prinzipiell möglich sei. Allerdings beobachteten sie stets die Bindung eines gewissen Anteils des doppelsträngigen PCR-Produktes an die stationäre Phase, was unweigerlich zu Ausbeuteverlusten führt. Darüber hinaus wird auch hier nicht die Abtrennung überschüssiger Mononukleotide, insbesondere dNTPs, beschrieben, so dass vor oder nach der Abtrennung der Primer noch zusätzliche Schritte zur Reinigung des PCR-Produktes notwendig sind.

Die vorliegende Erfindung ermöglicht dagegen die selektive Abtrennung einzelsträngiger Nukleinsäuren, umfassend unter anderem RNA und Oligonukleotid-Primer wie auch die Entfernung überschüssiger Mononukleotide in einem einzigen chromatographischen Schritt. Darüber hinaus können auch weitere in der Probe enthaltene Verunreinigungen, bspw. Salze, in diesem einen chromatographischen Schritt gleichzeitig abgetrennt werden. Die simultane Abtrennung von Mononukleotiden und einzelsträngigen Nukleinsäuren wird in der vorliegenden Erfindung dadurch erzielt, dass bevorzugt eine Kombination unterschiedlicher immobilisierter Metallionen verwendet wird, besonders bevorzugt eine Kombination eines weichen bzw. intermediären Metallions wie bspw. Ni²⁺ oder Cu²⁺ mit einem relativ harten Metallion wie bspw. Al³⁺ oder Fe³⁺. Dass auf diese Weise eine effiziente und sehr schnelle Reinigung von PCR-Produkten in einem einzigen chromatographischen Schritt erfolgen kann, ist umso überraschender, als dass gemäß dem Stand der Technik bisher davon ausgegangen werden musste, dass es bei der Verwendung harter Metallionen zu unspezifischen Wechselwirkungen der immobilisierten Metallionen mit dem Phosphatrückgrat der Nukleinsäuren kommt und in diesem Fall nicht nur einzelsträngige Nukleinsäuren und dNTPs, sondern auch doppelsträngige Nukleinsäuren, insbesondere doppelsträngige DNA, adsorbiert werden und somit eine selektive Entfernung einzelsträngiger Nukleinsäuren nicht mehr möglich ist (WO 02/46398).

Das poröse Chromatographie-Harz ist bevorzugt ein quervernetztes Silikat oder ein quervernetztes organisches Polymer, welches beim Vermischen mit Wasser, einer wässrigen Lösung oder einem wässrigen Puffer eine Matrix mit internen Poren definierter Größe bildet (Größenausschluss-Chromatographie-Harz). Bevorzugt ist das poröse Chromatographie-Harz ausgewählt aus der Gruppe enthaltend Dextrane, Agarose , Polyacrylamide, Propylencarbonat und Silikate oder Gemische derselben, welche bspw. unter den Handelsnamen Sephadex, Sephadex LH, Sepharose, Sephacryl, Superose, Superdex, Chromasolve, Chromasil und Nukleosil mit diversen Größenausschlussgrenzen (Quervernetzungsgraden) kommerziell erhältlich sind.

Bevorzugt weist das Größenausschluss-Chromatographie-Harz eine Größenausschlussgrenze auf, die kleine Moleküle wie bspw. Salze oder Peptide unterhalb eines Molekulargewichtes von 10⁴, bevorzugt unterhalb von 10³, weiter bevorzugt unterhalb von 10² Dalton (jeweils als globuläre Proteine) in die Poren eindringen lässt, während größere Moleküle (oberhalb des genannten Molekulargewichts) nicht in die Poren eindringen können und das Harz schneller passieren. Insbesondere doppelsträngige Nukleinsäure-Ketten mit mehr als 80, bevorzugt mehr als 100 bp sollten das Harz schnell passieren können, im Wesentlichen ohne in die Poren einzudringen. Dies lässt sich mit den kommerziell erhältlichen Harzen durch Routineversuche leicht bestimmen. Besonders bevorzugt wird erfindungsgemäß ein Sephadex-Material verwendet (bspw. von Sigma-Aldrich).

Entscheidend für eine effiziente und schnelle Reinigung doppelsträngiger Nukleinsäuren ist die Tatsache, dass die stationäre Phase sowohl interne Poren zur Entfernung von Molekülen mit einem geringen hydrodynamischen Radius und/oder Ionen, bspw. gelöste Salze aufweist, als auch immobilisierte Metallionen zur selektiven Bindung einzelsträngiger Nukleinsäuren und Mononukleotide umfasst. Die konkrete Anordnung dieser beiden Merkmale innerhalb der stationären Phase der erfindungsgemäßen Vorrichtung ist jedoch nicht von entscheidender Bedeutung für den Erfolg der chromatographischen Reinigung.

So umfasst die stationäre Phase in einer bevorzugten Ausführungsform der erfindungsgemäßen Vorrichtung zusätzlich zu dem wenigstens einen porösen Chromatographie-Harz (Größenausschluss-Chromatographie-Harz) wenigstens ein weiteres Material, an welchem die Metallionen immobilisiert vorliegen. Hierbei kann das wenigstens eine weitere Material jede Art von geeignetem Träger für die Metallkationen sein, ausgewählt aus teilchenförmigen Trägern wie beispielsweise Kügelchen, Pulver, Granulat, Kristalle oder ähnlichem, Membranen, gesinterten Partikeln jeder Form und Größe oder auch Fritten, Netzen, Fasern oder ähnlichen geeigneten Trägern. Das wenigstens eine weitere Material kann beispielsweise ausgewählt sein aus großporigen Zeoliten, Mordeniten oder ähnlichen, porösen Schäumen, porösen Harzen, Polymerkügelchen, einschließlich netzartiger Kügelchen, Oberflächen nicht-poröser, monolithischer Strukturen wie beispielsweise anorganischer monolithischer Strukturen, die bei katalytischen Umsetzungen verwendet werden, oder polymeren Strukturen, wie beispielsweise Epoxidharzen, oder auch aus Mischungen der genannten

Das Material ist bevorzugt ausgewählt aus üblichen Chromatographie-Harzen, wobei das Material, an dem die Metallionen immobilisiert sind dasselbe sein kann wie das Größenausschluss-Chromatographie-Harz oder sich von diesem unterscheiden kann, z.B. ein anderes poröses Harz sein kann, oder auch ein nicht-poröses Chromatographie-Harz.

Bevorzugt ist das Material, das als Träger für die Metallionen dient ausgewählt aus Polymerharzen, welche als für die Ligandenfunktionalisierung geeignet bekannt sind, einschließlich Sepharose, chemisch und / oder physikalisch modifizierte Sepharose, Agarose, chemisch und / oder physikalisch modifizierte Agarose, andere polymere Zucker oder chemisch und / oder physikalisch modifizierte Versionen davon, Cellulose, chemisch und / oder physikalisch modifizierte Cellulose, Polyolefine, chemisch und / oder physikalisch modifizierte Polyolefine, Polydiene, chemisch und / oder physikalisch modifizierte Polydiene, Polyurethane, chemisch und / oder physikalisch modifizierte Polyurethane, Polypeptid, chemisch und / oder physikalisch modifizierte Polypeptides, Polyamide, chemisch und / oder physikalisch modifizierte Polyamide, Polyalkylenoxide, chemisch und / oder physikalisch modifizierte Polyalkylenoxide wie z.B. Polyethylenglycole, chemisch und / oder physikalisch modifizierte Polyethylenglycole, Silicone, Elastomere, Thermoplastices, thermoplastische Elastomere oder andere polymere Substrate, ohne auf diese beschränkt zu sein.

Geeignete Membranen schließen permeable Membranen or semi-permeable Membranen, chemisch und/oder physikalisch modifizierte Membranen ein; geeignete anorganische Träger schließen Silicas, Silicate, Aluminiumoxide, Silica-Aluminiumoxide, Zeolite, Mordenite, Aluminate, Tonerden, oder jede andere Art von geeignetem anorganischen Träger mit ein; geeignete metallische Träger schließen Gold, Gold-Legierungen, Platin, Platin-Legierungen, Silber, Silber-Legierungen, Eisen, Eisen-Legierungen, wie bspw jede Art von Stahl, Kupfer, KupferLegierungen wie z.B. Messing oder Bronze, Zinn, Zinn-Legierungen, Aluminum, Aluminum-Legierungen, Silicium, Silicium-Legierungen und andere Halbleiter ein, jeweils ohne auf diese beschränkt zu sein.

Wird eine Kombination verschiedener Metallionen verwendet, so können diese gemeinsam an genau ein weiteres Material immobilisiert sein oder auch jeweils an unterschiedlichen Materialien immobilisiert sein, welche in einzelnen Schichten oder in Mischungen angeordnet sind.

In einer bevorzugten Ausführungsform sind das poröse Chromatographie-Harz und das zusätzliche Material mit den Metallionen in der chromatographischen Vorrichtung im Wesentlichen voneinander getrennt angeordnet, bevorzugt in Form zweier in Flussrichtung der mobilen Phase vom Einlass zum Auslass aufeinanderfolgenden Schichten, besonders bevorzugt dergestalt, dass das Material, an welchem die Metallionen immobilisiert sind, als obere Schicht und das Größenausschluss-Chromatographie-Harz als untere Schicht angeordnet ist. Eine derartige bevorzugte Ausführungsform ist bspw. in Abbildung 2 schematisch dargestellt. Hierbei folgen die Schichten direkt aufeinander, stehen also über mindestens eine Fläche in Kontakt, so dass die mobile Phase beim Austritt aus der einen Schicht unmittelbar in die andere Schicht eintritt und kein Umfüllen der partiell gereinigten Probe notwendig ist, wie es zum Beispiel erforderlich wäre, wenn eine immobilisierte Metallionen-Chromatographie nach oder vor einer Größenausschluss-Chromatographie in zwei getrennten chromatographischen Vorrichtungen ausgeführt werden würde.

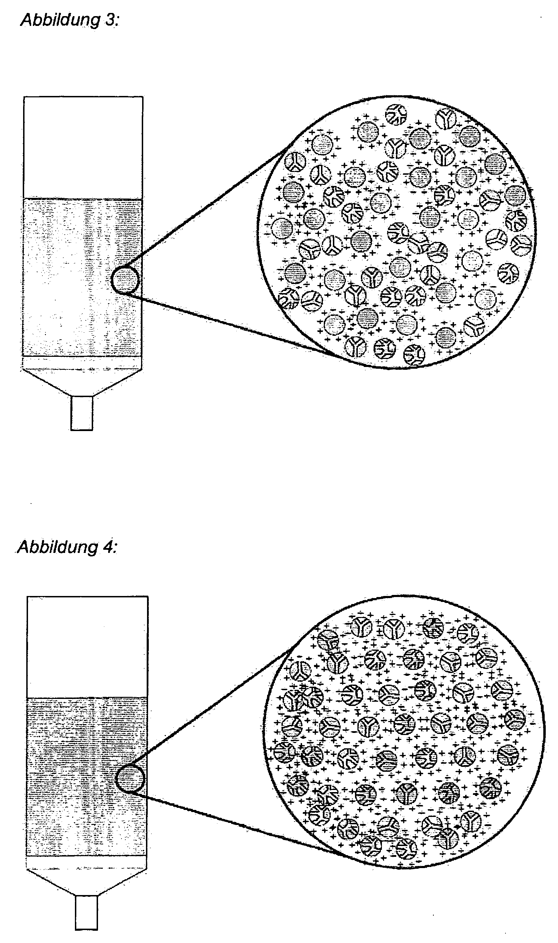

Alternativ können das poröse Chromatographie-Harz und das zusätzliche Material in einer weiteren bevorzugten Ausführungsform in der erfindungsgemäßen chromatographischen Vorrichtung weitgehend als homogene Mischung vorliegen (sogenanntes Mischbett-Harz/mixed-bed resin). Diese Ausführungsform ist schematisch in Abbildung 3 dargestellt.

In den beiden zuvor genannten Ausführungsformen kann das Material, auf dem die Metallionen immobilisiert vorliegen, entweder dasselbe sein wie das verwendete Chromatographie-Harz, oder sich von diesem unterscheiden. Im letzteren Fall wird bevorzugt eines der oben genannten Materialien verwendet, welches keine unspezifischen Wechselwirkungen mit der zu reinigenden Nukleinsäure eingeht.

Die Ausführungsformen gemäß Abb. 2 und 3 sind besonders bevorzugt.

In einer weiteren möglichen bevorzugten Ausführungsform sind die Metallionen an dem Größenausschluss-Chromatographie-Harz selbst immobilisiert. In diesem Fall ist ein weiterer Zusatz von unmodifiziertem Chromatographie-Harz nicht notwendig. Diese Ausführungsform ist in Abbildung 4 schematisch dargestellt.

Ferner kann die stationäre Phase auch ein poröses Chromatographie-Harz und mindestens ein zusätzliche Material umfassen, wobei die Metallionen sowohl an dem porösen Chromatographie-Harz als auch an dem mindestens einem zusätzlichen Material immobilisiert sind.

Insbesondere in einer Ausführungsform, bei der das poröse Chromatographie-Harz auch immobilisierte Metallionen trägt, ist es besonders bevorzugt, dass das Chromatographie-Harz ausgewählt ist aus quervernetzten Silikaten und/oder quervernetzten Polymeren, gewählt aus Dextranen, Agarose, Polyacrylamiden und Propylencarbonaten, oder Gemischen derselben, wobei bevorzugt deren Porengröße so definiert ist, dass kleine Moleküle wie bspw. Salze oder Peptide unterhalb eines Molekulargewichtes von 10⁴, bevorzugt unterhalb von 10³, weiter bevorzugt unterhalb von 10² Dalton (jeweils als globuläre Proteine) in die Poren eindringen können, während größere Moleküle (oberhalb des zuvor genannten Molekulargewichts) nicht in die Poren eindringen können und das Harz schneller passieren. Insbesondere doppelsträngige Nukleinsäure-Ketten mit mehr als 80, bevorzugt mehr als 100 bp sollten das Harz schnell passieren können, im Wesentlichen ohne in die Poren einzudringen. Besonders bevorzugt sind Dextrane, Polyacrylamide, Propylencarbonate und Silikate oder Gemische derselben.

In einer bevorzugten Ausführungsform umfasst die chromatographische Vorrichtung genau einen mit einer stationären Phase gefüllten Hohlraum und ist bevorzugt in Form einer Zentrifugationssäule (spin column) ausgebildet. Besonders bevorzugt ist die Vorrichtung so ausgestaltet, dass sie in ein übliches Reaktionsgefäß (wie beispielsweise ein Eppendorf-Cup) eingesetzt und das Eluat in einem solchen Reaktionsgefäß aufgefangen werden kann.

In einer weiteren bevorzugten Ausführungsform erlaubt die chromatographische Vorrichtung die parallele Isolierung und/oder Reinigung doppelsträngiger Nukleinsäuren aus unterschiedlichen Proben mithilfe einer einzigen Vorrichtung, wobei die chromatographische Vorrichtung mehrere, mit jeweils der stationären Phase gefüllte Hohlräume umfasst, welche in paralleler Weise angeordnet sind. In einer bevorzugten Ausführungsform ist eine solche Vorrichtung zur simultanen Isolierung und/oder Reinigung doppelsträngiger Nukleinsäuren aus unterschiedlichen Proben in Form einer Filterplatte angeordnet, die die Chromatographie durch Zentrifugation (Spin-Multiwellplatte) erlaubt.

Als Material für die chromatographische Vorrichtung können eine Vielzahl von aus dem Stand der Technik für derartige Vorrichtungen bekannten Materialien verwendet werden wie bspw. Glas, beschichteter Edelstahl, Kunststoffe etc., ohne darauf beschränkt zu sein. Bevorzugt besteht der Grundkörper aus einem Material, enthaltend ein organisches Polymer, bevorzugt ausgewählt aus der Gruppe bestehend aus Polyethylen (PE) und Polypropylen (PP)), Polycarbonat, Polystyrol, Polyphenysulfon oder Polysulfon. Die erfindungsgemäße Vorrichtung eignet sich zur chromatographischen Isolierung und/oder Reinigung doppelsträngiger Nukleinsäuren aus einer Mischung solcher Nukleinsäuren mit einzelsträngigen Nukleinsäuren, Oligonukleotiden wie bspw. überschüssigen Primem, Mononukleotiden wie dNTPs, Salzen und/oder sonstigen Verunreinigungen, die typischerweise in nukleinsäurehaltigen Proben, insbesondere in Proben nach erfolgter PCR vorliegen. Bevorzugt lässt sich die erfindungsgemäße Vorrichtung zur Isolierung und/oder Reinigung von Amplikationsprodukten einer PCR aus dem Reaktionsgemisch verwenden.

Die vorliegende Erfindung stellt weiterhin ein Verfahren zur chromatographischen Isolierung und/oder Reinigung doppelsträngiger Nukleinsäuren aus einer Mischung solcher Nukleinsäuren mit einzelsträngigen Nukleinsäuren, Oligonukleotiden, Mononukleotiden, Salzen und/oder sonstigen Verunreinigungen, bevorzugt zur Isolierung und/oder Reinigung von Amplifikationsprodukten einer PCR aus dem Reaktionsgemisch bereit, welches die folgenden Schritte umfasst: (1) Bereitstellen einer Probe enthaltend wenigstens doppelsträngige Nukleinsäuren in Form einer wässrigen Lösung; (2) Aufbringen dieser Lösung auf die dem Einlass zugewandte Seite der stationären Phase der erfindungsgemäßen chromatographischen Vorrichtung; (3) Eluieren der doppelsträngigen Nukleinsäuren und gleichzeitiges Auffangen des Eluates, wobei die Abtrennung der doppelsträngigen Nukleinsäuren von den weiteren in der Probe enthaltenen Bestandteilen in einem einzigen chromatographischen Schritt erfolgt.

Im Unterschied zu den bisher zur Reinigung doppelsträngiger Nukleinsäuren, insbesondere zur Reinigung der Amplifikationsprodukte einer PCR aus den Reaktionsgemischen eingesetzten chromatographischen Verfahren stellt das erfindungsgemäße Verfahren eine sog. "negative" Chromatographie dar, bei der nicht das zu reinigende Molekül an die stationäre Phase gebunden wird / von ihr zurückgehalten wird, sondern die Verunreinigungen (einzelsträngige DNA, RNA, Oligonukleotide wie Primer, Mononukleotide wie dNTPs, Salze etc.) mit der stationären Phase interagieren und dadurch zurückgehalten werden, während die doppelsträngige DNA gemeinsam mit dem in der Probe enthaltenem Wasser (der mobilen Phase) die stationäre Phase passiert. Als Elution doppelsträngiger Nukleinsäuren wird im Sinne der Erfindung daher der Schritt des Verfahrens bezeichnet, in welchem die doppelsträngigen Nukleinsäuren in dem mit der Probe aufgetragenen Lösungsmittelvolumen die stationäre Phase passieren. Im Sinne der Erfindung ist der Begriff Elution daher von der sogenannten desorptiven Elution der bind-wash-elute-Verfahren und auch der fraktionierenden Elution der klassischen Retentionschromatographie zu unterscheiden, bei welcher ein Vielfaches des Säulenvolumens eines geeigneten Lösungsmittels über eine stationäre Phase geleitet wird, um sukzessive die einzelnen Substanzen eines auf die stationäre Phase aufgetragenen Substanzgemisches in einzelnen Fraktionen zu erhalten. Das erfindungsgemäße chromatographische Verfahren ermöglicht eine Isolierung und/oder Reinigung doppelsträngiger Nukleinsäuren in einem einzigen chromatographischen Elutionsschtitt.

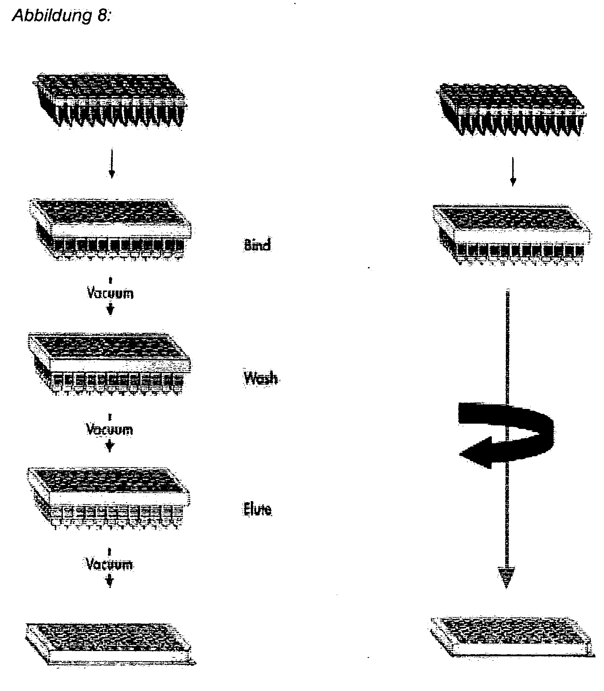

Hierbei können nicht nur lösliche Verunreinigungen entfernt werden, sondern die stationäre Phase der erfindungsgemäßen Vorrichtung wirkt auch als eine Art Filter für festes (ungelöstes) Material, bspw. für Niederschläge oder festes Material, welches bei einer Zelllyse zurückbleibt. Diese dringen nicht mit der Lösung in die stationäre Phase ein, sondern wird auf ihrer Oberfläche zurückgehalten. Hervorzuheben ist ferner die Tatsache, dass die Probe nach der Reinigung das annähernd gleiche Volumen und die annährend gleiche Konzentration an doppelsträngigen Nukleinsäuren besitzt wie vor der Reinigung, ohne dass zusätzliche Schritte zur Konzentrierung des Eluats (bspw. durch Fällung, Evoporation etc.) notwendig wären. Hierdurch werden potentielle Fehlerquellen, bspw. beim Umfüllen oder Umpipettieren, vermieden und Ausbeuteverluste minimiert. Ferner werden keine speziellen Puffer oder sonstige Lösungen zur Anbindung und Ablösung der zu reinigenden Substanzen benötigt, wodurch das Verfahren kostengünstiger und schneller als die üblicherweise zur Chromatographie von doppelsträngigen Nukleinsäuren verwendeten Verfahren ist. Dies wird durch Abbildung 8 verdeutlicht, in welcher die parallele Reinigung einer Vielzahl von Proben in einer Multiwellplatte unter Verwendung eines bind-wash-elute-Verfahrens (linke Spalte) und unter Verwendung des erfindungsgemäßen Verfahrens (rechte Spalte) dargestellt ist. Während in dem bind-wash-elute-Verfahren mindestens drei chromatographische Schritte erforderlich sind, erfolgt die Isolierung und Reinigung bei dem erfindungsgemäßen Verfahren in einem einzigen chromatographischen Schritt, bevorzugt durch Zentrifugation, symbolisiert durch den gebogenen Pfeil in Abb. 8.

Mit dem erfindungsgemäßen Verfahren können alle Arten doppelsträngiger Nukleinsäuren isoliert und/oder gereinigt werden wie doppelsträngige Ribonukleinsäuren (RNA), in denen beide Stränge des Doppelstrangs Ribonukleinsäuren sind, Doppelstränge aus jeweils einem Strang RNA und einem Strang DNA und doppelsträngige Nukleinsäuren, in welchen beide Stränge Desoxyribonukleinsäuren (DNA) darstellen. Die doppelsträngigen Nukleinsäuren sind nicht in ihrer Länge beschränkt und können bspw. Doppelstränge aus Oligonukleotiden mit relativ geringer Anzahl an Nukleotideinheiten darstellen, bspw. aus 5 bis 100 Mononukleotiden pro Strang aber auch Doppelstränge jeder beliebigen Anzahl von Basen, wie bspw. kurze bis langkettige PCR-Produkte (typischerweise 50bp bis mehrere kb) bis hin zu Plasmid-DNA und genomischer DNA (gDNA) (mehrere kb bis in den Mega-bp-Bereich, Bevorzugt stellen die doppelsträngigen Nukleinsäuren doppelsträngige Desoxyribonukleinsäuren (DNA) dar, besonders bevorzugt die als Amplifikationsprodukte einer PCR erhaltene lineare doppelsträngige DNA beliebiger Länge, bspw. von 50 bp bis 10 kb.

Bevorzugt stellt die Probe, aus welcher mit dem erfindungsgemäßen Verfahren die doppelsträngigen Nukleinsäuren isoliert und/oder gereinigt werden, das aus einer PCR ohne nachfolgende Reinigungsschritte erhaltene Reaktionsgemisch dar. So kann nach Beendigung der PCR das Reaktionsgemisch ohne nachfolgende Aufarbeitungs- und/oder Reinigungsschritte wie Verdünnung, Extraktion, Ausfällung oder sonstige üblicherweise zur Aufarbeitung und/oder Reinigung verwendete Verfahren direkt auf die erfindungsgemäße chromatographische Vorrichtung gegeben werden und mithilfe des erfindungsgemäßen Verfahrens in einem einzigen chromatographischen Schritt gereinigt werden, wobei hierbei sowohl einzelsträngige Nukleinsäuren, insbesondere ssDNA, sowie sämtliche niedermolekularen Bestandteile, bspw. Salze und dNTPs weitestgehend abgetrennt werden und sich im Eluat die gereinigte doppelsträngige DNA (das PCR-Produkt) befindet. Besonders vorteilhaft ist hierbei, dass die dsDNA in der nahezu gleichen Konzentration wie vor der Reinigung vorliegt und nicht während der Chromatographie verdünnt wurde. Dadurch sind in dem erfindungsgemäßen Verfahren keine zusätzlichen Schritte zur Konzentrierung der Probe aus dem Eluat notwendig, sondern das Eluat kann direkt nach der Chromatographie für nachfolgende Assays oder Analysen eingesetzt werden.

Die Stärke der nicht-kovalenten Bindung zwischen den immobilisierten Metallionen und den Stickstoffheterozyklen der Mononukleotide bzw. einzelsträngigen Nukleinsäuren ist unter anderem abhängig von dem pH-Wert der Probenlösung. Vorteilhafterweise wird die chromatographische Reinigung bei einem pH-Wert von ca. 4 bis 10 durchgeführt. Gegebenenfalls kann der pH-Wert einer Probe vor dem Aufbringen der Probenlösung auf die stationäre Phase der erfindungsgemäßen chromatographischen Vorrichtung durch Zugabe eines geeigneten Puffers eingestellt werden. Geeignete Puffer sind wasserbasierte Puffer, umfassend bspw. Goods-Puffer wie Mops, Mes und Hepes sowie wässrige Salzlösungen, bspw. von Alkalimetallacetaten oder -phosphaten. Substanzen, die eine hohe Affinität zu Metallionen haben, bspw. Tricin oder Citrat, werden als Bestandteile des Puffers bevorzugt vermieden.. Zur Einstellung des zur chromatographischen Reinigung geeigneten pH-Wertes wird die aus der PCR erhaltene Reaktionsmischung bevorzugt mit 1/20 bis 1/30 ihres Volumens an dieser Pufferlösung versetzt und anschließend auf die stationäre Phase der chromatographischen Vorrichtung gegeben.

Bevorzugt umfasst das Verfahren anschließend weiterhin einen Schritt des Inkubierens der auf die stationäre Phase aufgebrachten Lösung vor dem Eluieren der doppelsträngigen Nukleinsäuren. Durch die Inkubation, welche bevorzugt durch einfaches Stehenlassen der mit der Probenlösung beladenen Vorrichtung bei Raumtemperatur erfolgen kann, wird die selektive Anbindung der einzelsträngigen Nukleinsäuren und Mononukleotide an die immobilisierten Metallionen noch verbessert. Weder die Temperatur noch die Zeit der Inkubation unterliegen besonderen Beschränkungen und können bspw. bei 0 °C bis 80 °C für einen Zeitraum von wenigen Sekunden bis zu mehreren Stunden durchgeführt werden. Bevorzugt ist die Inkubation bei Raumtemperatur für einen Zeitraum von 0 Sekunden bis 20 Minuten, bevorzugt von 10 Sekunden bis 10 Minuten, weiterhin bevorzugt von 15. Sekunden bis 5 Minuten, insbesondere bevorzugt von 30 Sekunden bis 2 Minuten und ganz besonders bevorzugt von 1 Minute.

Das erfindungsgemäße Verfahren zeichnet sich unter anderem dadurch aus, dass das Volumen des erhaltenen Eluats dem Volumen der auf die stationäre Phase aufgebrachten Lösung der Probe vergleichbar ist. Bevorzugt weicht das Volumen des nach der chromatographischen Reinigung erhaltenen Eluats nicht mehr als +/- 50%, bevorzugt +/- 20 % von dem Volumen der auf die stationäre Phase aufgebrachten Probenlösung ab.

Die stationäre Phase in der chromatographischen Vorrichtung wird durch Einfüllen einer Suspension des Chromatographieharzes bzw. des Chromatographieharzes und des weiteren Materials erstellt. Bevorzugt wird beispielsweise eine Suspension mit ca. 30 - 70 %, beispielsweise mit ca. 50 % Feststoffanteil des (gequollenen) Harzes und / oder des weiteren Materials in (den) wenigstens einen Hohlraum des Grundkörpers gefüllt. Da es sich bei der Suspension um ein gequollenes Haz handelt, entspricht dies einem Feststoffanteil in der Suspension von ca. 3 bis 10 %, bevorzugt von ca. 5 bis 7 %. Bei Verwendung eines üblichen, in ein 1,5 ml Eppendorf-Cup passenden Spin-Säulchens als chromatographische Vorrichtung besteht die Gesamteinwaage der Suspension aus ca. 600 bis 700 mg, entsprechend ungefähr 600 bis 700 µg der Suspension.

Die Probe enthaltend die zu isolierenden und/oder reinigenden doppelsträngigen Nukleinsäuren wird als wässrige Lösung (mobile Phase) auf die Oberfläche der stationären Phase gegeben und passiert dann aufgrund der Schwerkraft oder durch Anlegen von Über- oder Unterdruck oder unter Einwirkung von Zentrifugalkräften, ohne hierauf beschränkt zu sein, die stationäre Phase (Elution). Zum Eluieren der doppelsträngigen Nukleinsäuren ist außer dem Volumen, welches in der aufgetragenen Probe enthalten ist, keine Zugabe zusätzlichen Lösungsmittels erforderlich. Bevorzugt erfolgt das Eluieren der doppelsträngigen Nukleinsäuren durch Anlegen einer Zentrifugalkraft an die chromatographische Vorrichtung. In diesem Fall ist die chromatographische Vorrichtung bevorzugt in Form einer Spin-Säule geeigneter Größe oder einer in eine Zentrifuge einsetzbaren Filterplatte ausgebildet. Bevorzugt wird hierzu die chromatographische Vorrichtung 1 Sekunde bis 20 Minuten, besonders bevorzugt 10 Sekunden bis 10 Minuten und insbesondere 30 Sekunden bis 1 Minute zentrifugiert. Die angelegte Zentrifugalkraft beträgt bevorzugt 30 bis 3.000 g, besonders bevorzugt 300 bis 2.000 g und insbesondere 750 g.

Die erfindungsgemäße chromatographische Vorrichtung kann entweder als vorgepackte chromatographische Vorrichtung vorliegen, in welcher die stationäre Phase bereits in dem Hohlraum der chromatographischen Vorrichtung als Suspension in einem geeigneten Lösungsmittel vorliegt, bevorzugt als Suspension in einem wässrigen Puffer, welcher vor dem Aufbringen der zu reinigenden Probe durch Gravitation, Über- oder Unterdruck und bevorzugt durch Anlegen einer Zentrifugalkraft unter Ausbildung der zur Chromatographie geeigneten Matrix entfernt wird.

Alternativ kann die stationäre Phase auch in trockenem Zustand in der chromatographische Vorrichtung enthalten sein oder die chromatographischen Materialien, welche die stationäre Phase bilden, können separat von der chromatographischen Vorrichtung bereitgestellt werden. In diesem Fall umfasst das erfindungsgemäße Verfahren weiterhin einen Schritt zur Herstellung der Gel-Matrix durch Suspendieren der chromatographischen Materialien in Wasser, einer wässrigen Lösung oder einem wässrigen Puffer, Quellenlassen der Materialien in der Suspension unter Ausbildung der Gel-Matrix und Entfernen des überschüssigen Wassers, wässrigen Lösungsmittels oder wässrigen Puffers durch Zentrifugieren der chromatographischen Vorrichtung, enthaltend das gequollene Harz vor dem Aufbringen der Probenlösung auf die stationäre Phase. Das Chromatographie-Harz kann hierbei vor oder nach dem Quellenlassen durch Wasser, eine wässrige Lösung oder einen wässrigen Puffer in die chromatographische Vorrichtung eingefüllt werden.

Die Erfindung umfasst weiterhin einen Kit zur Isolierung und/oder Reinigung von doppelsträngigen Nukleinsäuren, bevorzugt von doppelsträngiger DNA, umfassend die erfindungsgemäße chromatographische Vorrichtung oder deren Ausgangsmaterialien, um gemäß dem vorherigen Absatz die chromatographische Vorrichtung zum gebrauch herzurichten und ggf. eine Pufferlösung zur Einstellung des pH-Wertes der Probenlösung vor der chromatographischen Reinigung. Die Pufferlösung ist bevorzugt ausgewählt aus der Gruppe enthaltend Mops, Mes, Hepes und wässrige Lösungen von Alkalimetallacetaten oder-phosphaten, und ist besonders bevorzugt eine wässrige Natriumacetat-Lösung.

### Beschreibung der Abbildungen

Abbildung 1 ist eine schematische Darstellung der erfindungsgemäßen chromatographischen Vorrichtung mit einem Grundkörper (1), welcher in seinem Inneren einen Hohlraum definiert, in welchem sich eine stationäre Phase (4) befindet und der weiterhin mit einem Einlass (2), einem Auslass (3) und einer porösen Fritte (5) versehen ist, welche die stationäre Phase in dem Hohlraum zurückhält, sowie einem Deckel (6) als Verschlussvonichtung zum Verschließen des Einlasses (2) des Hohlraumes.

Abbildung 2 zeigt eine bevorzugte Ausführungsform der in dem Hohlraum enthaltenen stationären Phase, in welcher das Material, an welchem die Metallionen immobilisiert sind und das poröse Größenausschluss-Chromatographie-Harz in Form zweier in Flussrichtung der mobilen Phase vom Einlass zum Auslass aufeinander folgenden, getrennten Schichten angeordnet sind. Die in Flussrichtung obere Schicht ist in dieser Abbildung das chromatographische Material, an welches die Metallionen immobilisiert sind. Dieses ist in Abbildung 2 durch Kügelchen dargestellt, welche von positiven Ladungen (symbolisierte durch "+") umgeben sind. Das poröse Größenausschluss-Chromatographie-Harz ist hier in Flussrichtung als untere Schicht angeordnet und durch Kügelchen mit internen Kanälen dargestellt.

Abbildung 3 zeigt eine weitere bevorzugte Ausführungsform der stationären Phase der erfindungsgemäßen chromatographischen Vorrichtung, in welcher die stationäre Phase als weitgehend homogene Mischung (sog. Mischbett-Harz/mixed-bed resin) vorliegt. Die symbolische Darstellung der unterschiedlichen Chromatographie-medien ist wie in Abbildung 2 erläutert.

Abbildung 4 zeigt eine weitere bevorzugte Ausführungsform der stationären Phase der erfindungsgemäßen chromatographischen Vorrichtung, in welcher die Metallionen an dem Größenausschluss-Chromatographie-Harz selbst immobilisiert sind, verdeutlicht durch die positiven Ladungen, welche die Kügelchen mit internen Kanälen umgeben.

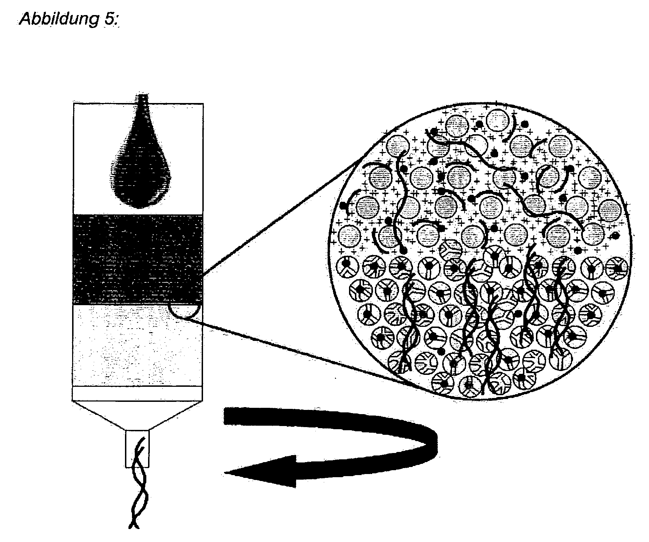

Abbildung 5 ist eine schematische Darstellung der chromatographischen Reinigung unter Verwendung der erfindungsgemäßen Vorrichtung. In der dargestellten Ausführungsform sind das poröse Größenausschluss-Chromatographie-Harz und das Material, welches die immobilisierten Metallionen enthält, in zwei getrennten Schichten angeordnet. Die aufgetragene Probe passiert beim Zentrifugieren (symbolisiert durch den gebogenen Pfeil) zunächst das Material, an welchem die Metallionen immobilisiert sind. Durch nicht-kovalente Wechselwirkungen werden hierbei einzelsträngige Nukleinsäuren (symbolisiert durch einzelne schwarze Schlangenlinien) und dNTPs an diesem Material zurückgehalten, während die doppelsträngigen Nukleinsäuren (dargestellt durch jeweils zwei miteinander verschlungene schwarze Schlangenlinien) und sonstige Verunreinigungen wie Salze (dargestellt durch kleine, ausgefüllte Kreise) diese erste chromatographische Schicht passieren können. Unmittelbar auf diese erste Schicht erfolgt die zweite chromatographische Schicht, welche durch das poröse Größenausschluss-Chromatographie-Harz gebildet wird. Niedermolekulare Bestandteile wie Salze dringen in die Poren des Größenausschluss-Chromatographie-Harzes ein und werden in diesem zurückgehalten, während die doppelsträngigen Nukleinsäuren aufgrund ihrer Größe bzw. ihres hydrodynamischen Volumens nicht in die Poren eindringen können und somit mit dem entsalzten Lösungsmittel aus dem Auslass der chromatographischen Vorrichtung in gereinigter Form austreten.

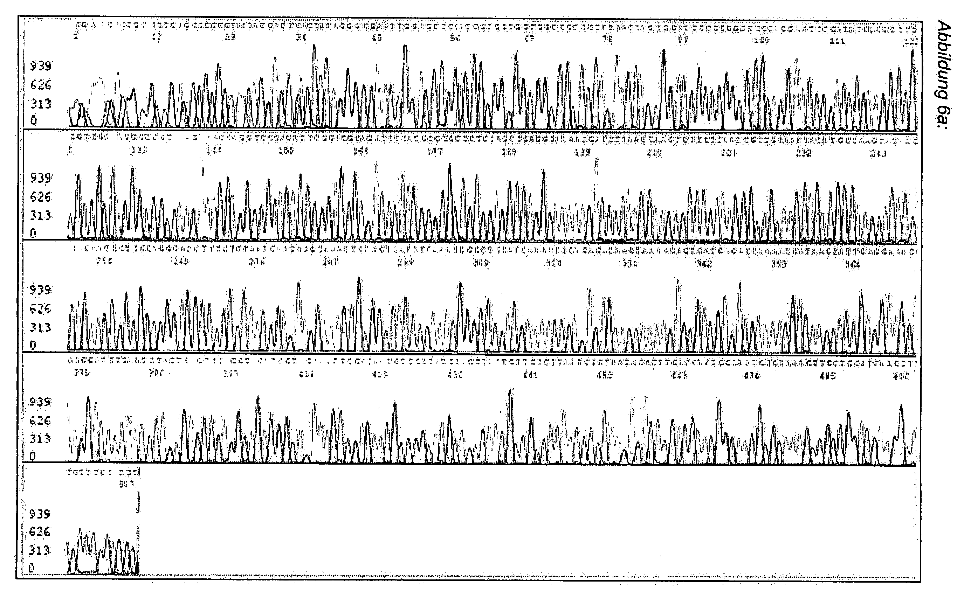

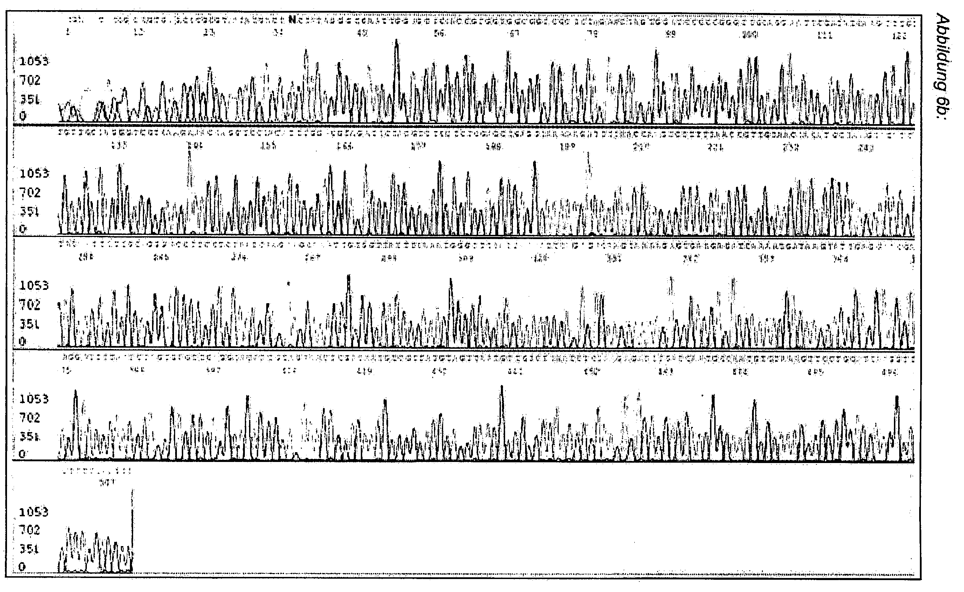

Die Abbildungen 6a und 6b zeigen das Ergebnis der Sequenzierung eines aus einer PCR erhaltenen Reaktionsgemisches, welches vor der Aufreinigung mit 30 µmol dNTPs versetzt wurde und anschließend über eine Sephadex-Spin-Säule (Abbildung 6a) gereinigt wurde beziehungsweise über eine erfindungsgemäße Spin-Säule, welche eine Kombination aus einem Sephadex-Material und immobilisierten Cu²⁺ und Al³-lonen (Abbildung 6b) enthielt.

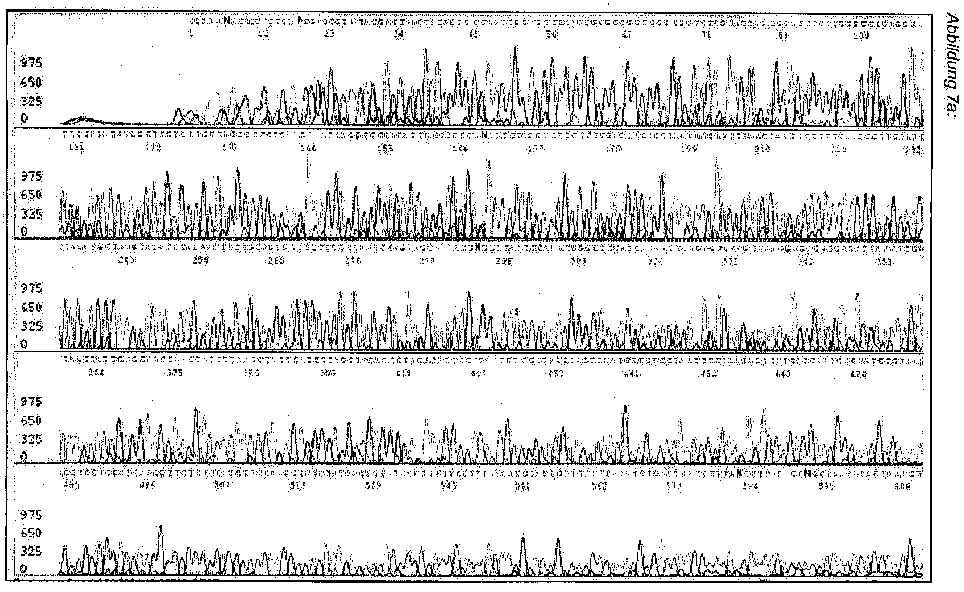

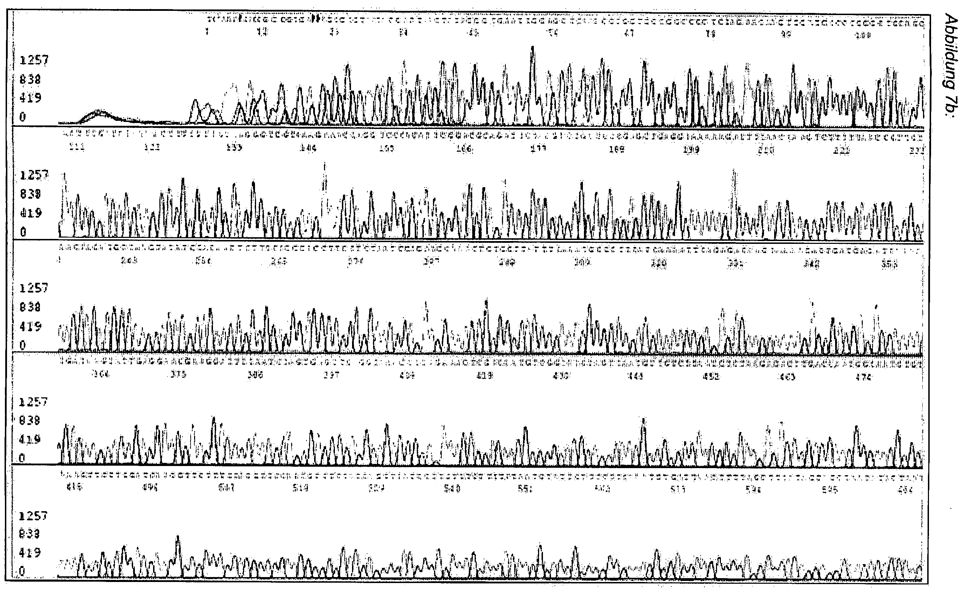

Die Abbildungen 7a und 7b zeigen das Ergebnis der Sequenzierung eines aus einer PCR erhaltenen Reaktionsgemisches, welches vor der Aufreinigung mit 100 pmol reversen Primern versetzt wurde und anschließend über eine Sephadex-Spin-Säule (Abbildung 7a) gereinigt wurde beziehungsweise über eine erfindungsgemäße Spin-Säule, welche eine Kombination aus einem Sephadex-Material und immobilisierten Cu²⁺ und Al³-Ionen (Abbildung 7b) enthielt:

Abbildung 8 zeigt eine Gegenüberstellung der notwendigen Schritte bei der chromatographischen Reinigung nukleinsäurehaltiger Proben unter Verwendung von a) Adsorptionsmaterialien, welche eine sogenannte Bind-Wash-Elute-Routine erfordern (linke Seite) und b) einer erfindungsgemäßen chromatischen Vorrichtung in Form einer sog. Spin-Multiwellplatte.

### Beispiele

### Beispiel 1: Herstellung einer erfindungsgemäßen Spin-Säule

Ca. 7g Sephadex G50 Feststoff (GE Healthcare, München, Deutschland) wurden mehrere Stunden in in 100 mg dH₂O bzw. 0,02% Natriumazid-Lösung gequollen (Ca 7% w/v bzw. ca. 50% v/v), und in eine kommerziell erhältliche Filtrationssäule mit Fritte eingefüllt (Qiagen Hilden, Deutschland) und mit 300 µl 50 % (v/v) Cu²⁺/Al³⁺-lda-Agarose (IDA-Superflow 6 Suspension (QIAGEN, Hilden, Deutschland) wurde mit einem Überschuss einer 250mM Lösung der entsprechenden Ionen gemischt und über Nacht bei Raumtemperatur geschüttelt. Danach wurde zehnmal mit dH₂O gewaschen und die Suspension in dem entsprechenden Bindepuffer auf 50% v/v eingestellt) überschichtet. Durch Zentrifugation bei 1.000 g für 5 min wurde das überschüssige zur Quellung verwendete Lösungsmittel entfernt und die Gel-Matrix zur Chromatographie vorbereitet.

### Beispiel 2: Reinigung von doppelsträngigen Nukleinsäuren aus einem PCR-Gemisch

25 µl des aus einer PCR erhaltenen Reaktionsgemisches wurden mit 1 µl 3 M NaAc (aq, pH 5.5) versetzt und auf die dem Einlass zugewandte Seite der stationären Phase einer unter Beispiel 1 beschriebenen Spin-Säule gegeben. Die beladene Säule wurde 1 min bei Raumtemperatur stehen gelassen und anschließend 5 min bei 1.000 g zentrifugiert, wobei das Eluat in einem geeigneten Auffangröhrchen aufgefangen wurde. Das Elutionsvolumen war dem Ausgangsprobenvolumen von etwa 25 µl vergleichbar.

### Beispiel 3: Entfernung überschüssiger dNTPs und Primer

Zur Verdeutlichung des Reinigungseffektes der erfindungsgemäßen chromatographischen Vorrichtungen wurden je 25 µl eines aus einer PCR erhaltenen Reaktionsgemisches mit je 30 µmol dTNPs (Endkonzentration) bzw. je 100 pmol reverser Primer versetzt und anschließend mit Hilfe einer kommerziell erhältlichen Sephadex-Spin-Säule (QIAGEN, Hilden, Deutschland) beziehungsweise der erfindungsgemäßen chromatographischen Vorrichtung wie unter Beispiel 2 beschrieben gereinigt. Die erhaltenen Eluate wurden anschließend jeweils durch Sequenzierung analysiert. Die Ergebnisse sind in den Abbildungen 6a bis 7b dargestellt.

Aus den Abbildungen 6a (Reinigung mit Sephadex) und 6b (Reinigung mit der erfindungsgemäßen Vorrichtung) ist deutlich zu erkennen, dass das Elektropherogramm derjenigen Probe, welche mit der erfindungsgemäßen Vorrichtung gereinigt wurde, durch deutlich schärfere Signale (Peaks) und wenig bis gar kein Hintergrundrauschen gekennzeichnet ist. Im Vergleich zu der mit der kommerziell erhältlichen Sephadex-Säule gereinigten Probe ist die mit der erfindungsgemäßen Vorrichtung gereinigten Probe dem Elektropherogramm nach von deutlich höherer Qualität, was zeigt, dass auch ein großer Überschuss nicht eingebauter dNTPs effektiv mit der erfindungsgemäßen Vorrichtung entfernt werden kann.

Der gleiche Trend ist in den Abbildungen 7a und 7b zu erkennen, die das Elektropherogramm einer Sequenzierungsreaktion zweier PCR-Proben zeigen, von denen die eine unter Verwendung einer kommerziell erhältlichen Sephadex-Spin-Säule (Abbildung 7a) und die andere unter Verwendung der erfindungsgemäßen Vorrichtung (Abbildung 7b) gereinigt wurde, nachdem beide zuvor mit jeweils 100 µmol reversen Primern versetzt wurden.

## Patentansprüche

1. Chromatographische Vorrichtung zur Isolierung und/oder Reinigung von doppelsträngigen Nukleinsäuren, bevorzugt von doppelsträngiger DNA, umfassend:
1. einen Grundkörper (1),
2. mindestens einen Hohlraum innerhalb des Grundkörpers, welcher mit einem Einlass (2) und einem Auslass (3) versehen ist,
3. eine stationäre Phase (4), welche sich in dem Hohlraum befindet,
**dadurch gekennzeichnet, dass** die stationäre Phase (4) wenigstens mindestens ein poröses Chromatographie-Harz, welches als Größenausschluss-Chromatographie-Medium fungiert, und immobilisierte Metallionen umfasst.

2. Vorrichtung gemäß Anspruch 1, weiterhin umfassend mindestens eines der folgenden Merkmale: einen porösen Filter (5), eine poröse Fritte (5) oder eine Membran (5), der/die zwischen dem Auslass (3) des Hohlraums und der stationären Phase (4) angeordnet ist und die stationäre Phase (4) in dem Hohlraum zurückhält, mindestens eine Verschlussvorrichtung (6) zum Verschließen des Einlasses (2) und/oder des Auslasses (3) des Hohlraumes, mindestens ein Auffanggefäß zum Auffangen der nach erfolgter Elution aus dem Auslass (3) austretende mobile Phase (Eluat).

3. Vorrichtung gemäß einem der Ansprüche 1 oder 2, **dadurch gekennzeichnet, dass** die immobilisierten Metallionen zwei-, drei- oder vierwertige Metallkationen oder Mischungen davon sind, bevorzugt ausgewählt aus der Gruppe bestehend aus Zn²⁺, Co²⁺, Cu²⁺, Ni²⁺, Fe²⁺, Mn²⁺, Al³⁺ Co³⁺, Ga³⁺ und Zr⁴⁺ oder eine Kombination derselben, besonders bevorzugt wenigstens ein zweiwertiges Metallkation, ausgewählt aus der Gruppe bestehend aus Zn²⁺, Co²⁺, Cu²⁺ und Ni²⁺, optional in Kombination mit Al³⁺-Ionen, insbesondere Cu²⁺-Ionen, optional in Kombination mit Al³⁺-Ionen.

4. Vorrichtung gemäß einem der Ansprüche 1 bis 3, **dadurch gekennzeichnet, dass** die Metallionen mit Hilfe wenigstens eines Linkers an wenigstens einen Teil der stationären Phase immobilisiert sind, wobei der Linker bevorzugt die allgemeine Formel A-R-Z aufweist, in der A eine Ankergruppe zur kovalenten Anbindung des Linkers an wenigstens einen Teil der stationären Phase darstellt, Z eine mehrzähnige Kopfgruppe zur Chelatisierung der Metallionen und R eine lineare, verzweigte oder zyklische Kohlenwasserstoffkette darstellt, welche die Ankergruppe A und die Kopfgruppe Z verbindet und die substituiert oder unsubstituiert sein kann und in der eine oder mehrere Kohlenstoffatome durch Heteroatome ausgetauscht sein können und bevorzugt ausgewählt ist aus der Gruppe bestehend aus Iminodiessigsäure (IDA), Nitrilotriessigsäure (NTA), *N*-carboxymethylierte Asparaginsäure (CM-Asp) und Tris-(2-ethylaminoethyl)amin (TREN).

5. Vorrichtung gemäß einem der Ansprüche 1 bis 4, **dadurch gekennzeichnet, dass** das poröse Chromatographie-Harz ein quervernetztes Silikat oder ein organisches Polymer ist, welches beim Vermischen mit Wasser, einer wässrigen Lösung oder einem wässrigen Puffer eine Gel-Matrix mit internen Poren definierter Größe bildet (Größenausschluss-Chromatographie-Harz), bevorzugt ausgewählt aus der Gruppe enthaltend Dextrane, Agarose und Polyacrylamide oder Gemische derselben.

6. Vorrichtung gemäß einem der Ansprüche 1 bis 5, **dadurch gekennzeichnet, dass** die stationäre Phase (4) zusätzlich zu dem wenigstens einem porösen Chromatographie-Harz (Größenausschluss-Chromatographie-Harz) wenigstens ein weiteres Material umfasst, an welchem die Metallionen immobilisiert vorliegen.

7. Vorrichtung gemäß Anspruch 6, **dadurch gekennzeichnet, dass**
a) das poröse Chromatographie-Harz und das zusätzliche Material in der chromatographischen Vorrichtung
i) im wesentlichen voneinander getrennt angeordnet sind, bevorzugt in Form zweier in Flussrichtung der mobilen Phase vom Einlass (2) zum Auslass (3) aufeinander folgenden Schichten, besonders bevorzugt dergestalt, dass das Material, an welches die Metallionen immobilisiert sind, als obere Schicht und das Größenausschluss-Chromatographie-Harz als untere Schicht angeordnet ist, oder
ii) weitgehend als homogene Mischung vorliegen, oder
b) die Metallionen an dem Größenausschluss-Chromatographie-Harz selbst immobilisiert sind.

8. Vorrichtung gemäß einem der Ansprüche 1 bis 7, **dadurch gekennzeichnet, dass** die chromatographische Vorrichtung
i) genau einen mit einer stationären Phase gefüllten Hohlraum (2) umfasst, und die chromatographische Vorrichtung bevorzugt in Form einer Zentrifugationssäule (spin column) ausgebildet ist, oder
ii) zur parallelen Isolierung und/oder Reinigung doppelsträngiger Nukleinsäuren aus unterschiedlichen Proben mehrere, mit jeweils der stationären Phase (4) gefüllte Hohlräume umfasst, welche in paralleler Weise angeordnet sind, bevorzugt in Form einer Filterplatte.

9. Vorrichtung gemäß einem der Ansprüche 1 bis 8, **dadurch gekennzeichnet, dass** der Grundkörper aus einem Material enthaltend ein organisches Polymer besteht, bevorzugt ausgewählt aus der Gruppe bestehend aus Polyethylen (PE) Polypropylen (PP), Polycarbonat, Polystyrol, Polyphenylsulfon oder Polysulfon.

10. Verwendung einer Vorrichtung gemäß einem der Ansprüche 1 bis 9 zur chromatographischen Isolierung und/oder Reinigung doppelsträngiger Nukleinsäuren aus einer Mischung solcher Nukleinsäuren mit einzelsträngigen Nukleinsäuren, Oligonukleotiden, Mononukleotiden, Salzen und/oder sonstigen Verunreinigungen, bevorzugt zur Isolierung und/oder Reinigung von Amplifikationsprodukten einer PCR aus dem Reaktionsgemisch.

11. Verfahren zur chromatographischen Isolierung und/oder Reinigung doppelsträngiger Nukleinsäuren aus einer Mischung solcher Nukleinsäuren mit einzelsträngigen Nukleinsäuren, Oligonukleotiden, Mononukleotiden, Salzen und/oder sonstigen Verunreinigungen, bevorzugt zur Isolierung und/oder Reinigung von Amplifikationsprodukten einer PCR aus dem Reaktionsgemisch, umfassend die folgenden Schritte:
1. Bereitstellen einer Probe enthaltend wenigstens doppelsträngige Nukleinsäuren in Form einer wässrigen Lösung,
2. Aufbringen dieser Lösung auf die dem Einlass zugewandte Seite der stationären Phase einer chromatographischen Vorrichtung gemäß einem der Ansprüche 1 bis 9,
3. Eluieren der doppelsträngigen Nukleinsäuren und gleichzeitiges Auffangen des Eluats,
**dadurch gekennzeichnet, dass** die Abtrennung der doppelsträngigen Nukleinsäuren von den weiteren in der Probe enthaltenen Bestandteilen in einem chromatographischen Schritt erfolgt.

12. Verfahren gemäß Anspruch 11, **dadurch gekennzeichnet, dass** die doppelsträngigen Nukleinsäuren doppelsträngige Desoxyribonukleinsäuren (DNA) darstellen, bevorzugt die als Amplifikationsprodukte einer PCR erhaltene lineare doppelsträngige DNA

13. Verfahren gemäß einem der Ansprüche 11 oder 12, **dadurch gekennzeichnet, dass** das Eluieren der doppelsträngigen Nukleinsäuren durch Anlegen einer Zentrifugalkraft an die chromatographische Vorrichtung erfolgt.

14. Verfahren gemäß Anspruch 13, weiterhin umfassend einen Schritt zur Herstellung der Gel-Matrix im Hohlraum der chromatographischen Vorrichtung durch Suspendieren eines porösen Chromatographie-Harzes in Wasser, einer wässrigen Lösung oder einem wässrigen Puffer, Quellenlassen des Harzes in der Suspension unter Ausbildung der Gel-Matrix und Entfernen des überschüssigen Wasser, wässrigen Lösungsmittels oder wässrigen Puffers durch Zentrifugieren der chromatographischen Vorrichtung enthaltend das gequollene Harz vor dem Aufbringen der Probenlösung auf die stationäre Phase.

15. Kit zur Isolierung und/oder Reinigung von doppelsträngigen Nukleinsäuren, bevorzugt von doppelsträngiger DNA, umfassend eine chromatographische Vorrichtung gemäß einem der Ansprüche 1 - 10 oder deren Ausgangsmaterialien und ggf. eine Pufferlösung zur Einstellung des pH-Wertes der Probenlösung vor der chromatographischen Reinigung, wobei die Pufferlösung bevorzugt ausgewählt ist aus der Gruppe enthaltend Mops, Mes, Hepes und wässrige Lösungen von Alkalimetallacetaten oder -phosphaten, bevorzugt eine wässrige Natriumacetat-Lösung.

## Claims

1. A chromatographic device for isolating and/or cleaning double-stranded nucleic acids, preferably double-stranded DNA, comprising:
1. a base body (1);
2. at least one hollow space inside the base body, which is equipped with an inlet (2) and an outlet (3);
3. a stationary phase (4), which is arranged in the hollow space;
**characterised in that** the stationary phase (4) comprises at least one porous chromatographic resin, which operates as a size exclusion chromatographic medium, and comprises immobilised metal ions.

2. The device in accordance with Claim 1, further comprising at least one of the following features: a porous filter (5), a porous frit (5) or a membrane (5), which is disposed between the outlet (3) of the hollow space and the stationary phase (4) and retains the stationary phase (4) in the hollow space, at least one locking device (6) for locking the inlet (2) and/or the outlet (3) of the hollow space, at least one collecting vessel for collecting the mobile phase (eluate) seeping out of the outlet (3) once elution has been completed.

3. The device in accordance with one of Claims 1 or 2, **characterised in that** the immobilised metal ions are bivalent, trivalent or tetravalent metal cations or mixtures thereof, preferably selected from the group consisting of Zn²⁺, Co²⁺, Cu²⁺, Ni²⁺, Fe²⁺, Mn²⁺, Al³⁺, Co³⁺, Ga³⁺ and Zr⁴⁺ or a combination of the same, especially preferably at least one bivalent metal cation, selected from the group consisting of Zn²⁺, Co²⁺, Cu²⁺ und Ni²⁺, optionally in combination with Al³⁺ ions, in particular Cu²⁺ ions, optionally in combination with Al³⁺ ions.

4. The device in accordance with one of Claims 1 to 3, **characterised in that** the metal ions are immobilised with the aid of at least one linker on at least one part of the stationary phase, wherein the linker preferably follows the general formula A-R-Z, in which A represents an anchor group for covalent linking of the linker to at least one part of the stationary phase, Z a polydentate head group for chelating the metal ions, and R a linear, branched or cyclic hydrocarbon chain, which connects the anchor group A and the head group Z and which may be substituted or unsubstituted and in which one or more carbon atoms can be exchanged by heteroatoms, and is preferably selected from the group consisting of iminodiacetic acid (IDA), nitrilotriacetic acid (NTA), *N*-carboxymethylated aspartic acid (CM-Asp) and tris (2-ethylaminoethyl)amine (TREN).

5. The device in accordance with one of Claims 1 to 4, **characterised in that** the porous chromatographic resin is a cross-linked silicate or an organic polymer which, when mixed with water, an aqueous solution or an aqueous buffer, forms a gel matrix with internal pores of a defined size (size exclusion chromatographic resin), preferably selected from the group containing dextrane, agarose and polyacrylamides or mixtures of the same.

6. The device in accordance with one of Claims 1 to 5, **characterised in that**, in addition to said at least one porous chromatographic resin (size exclusion chromatographic resin), the stationary phase (4) comprises at least one further material, on which the metal ions are present in an immobilised state.

7. The device in accordance with Claim 6, **characterised in that**
a) the porous chromatographic resin and the additional material in the chromatographic device
i) are disposed substantially apart from one another, preferably in the form of two consecutive layers in the direction of flow of the mobile phase from the inlet (2) to the outlet (3), especially preferably formed in such a way that the material on which the metal ions are immobilised is arranged as the upper layer and the size exclusion chromatographic resin as the lower layer; or
ii) largely form a homogeneous mixture; or
b) the metal ions are immobilised on the size exclusion chromatographic resin itself.

8. The device in accordance with one of Claims 1 to 7, **characterised in that** the chromatographic device
i) comprises exactly one hollow space (2) filled with a stationary phase, and the chromatographic device is preferably designed in the shape of a centrifugal column (spin column); or
ii) for the purpose of parallel isolation and/or cleaning comprises double-stranded nucleic acids from different samples, comprises several hollow spaces each filled with the stationary phase (4), which are arranged in parallel, preferably in the form of a filter plate.

9. The device in accordance with one of Claims 1 to 8, **characterised in that** the base body consists of a material containing an organic polymer, preferably selected from the group consisting of polyethylene (PE), polypropylene (PP), polycarbonate, polystyrene, polyphenylsulphone or polysulphone.

10. Use of a device in accordance with one of Claims 1 to 9 for chromatographic isolation and/or cleaning of double-stranded nucleic acids from a mixture of such nucleic acids with single-stranded nucleic acids, oligonucleotides, mononucleotides, salts and/or any other impurities, preferably for the isolation and/or cleaning of amplification products of a PCR from the reaction mixture.

11. A method of chromatographic isolation and/or cleaning of double-stranded nucleic acids from a mixture of such nucleic acids with single-stranded nucleic acids, oligonucleotides, mononucleotides, salts and/or any other impurities, preferably for the isolation and/or cleaning of amplification products of a PCR from the reaction mixture, comprising the following steps:
1. Providing a sample containing at least double-stranded nucleic acids in the form of an aqueous solution;
2. applying this solution to the side of the stationary phase of a chromatographic device in accordance with one of Claims 1 to 9 facing the inlet;
3. eluting the double-stranded nucleic acids and simultaneously collecting the eluate, **characterised in that** the separation of the double-stranded nucleic acids from the other components contained in the sample is undertaken in a chromatographic step.

12. The method in accordance with Claim 11, **characterised in that** the double-stranded nucleic acids constitute double-stranded deoxyribonucleic acids (DNA), preferably the linear double-stranded DNA contained as amplification products of a PCR.

13. The method in accordance with one of Claims 11 or 12, **characterised in that** the elution of the double-stranded nucleic acids is performed by applying a centrifugal force to the chromatographic device.

14. The method in accordance with Claim 13, furthermore comprising a step to manufacture the gel matrix in the hollow space of the chromatographic device by suspending a porous chromatographic resin in water, an aqueous solution or an aqueous buffer, leaving the resin to steep in the suspension while forming the gel matrix and removing the superfluous water, aqueous solution or aqueous buffer through centrifugation of the chromatographic device containing the steeped resin prior to applying the sample solution to the stationary phase.

15. A kit for isolating and/or cleaning double-stranded nucleic acids, preferably double-stranded DNA, comprising a chromatographic device in accordance with one of Claims 1 - 10 or the raw materials of the latter and, if applicable, a buffer solution to set the pH value of the sample solution prior to the chromatographic cleaning, wherein the buffer solution is preferably selected from the group containing Mops, Mes, Hepes and aqueous solutions of alkaline metal acetates or phosphates, preferably an aqueous sodium acetate solution.

## Revendications

1. Dispositif de chromatographie pour l'isolement et/ou la purification d'acides nucléiques double brin, de préférence d'ADN double brin, comprenant :
1. un corps de base (1),
2. au moins un espace creux à l'intérieur du corps de base, lequel est pourvu d'une entrée (2) et d'une sortie (3),
3. une phase stationnaire (4), laquelle se situe dans l'espace creux,
**caractérisé en ce que** la phase stationnaire (4) comprend au moins au moins une résine poreuse de chromatographie, laquelle fait fonction de milieu de chromatographie d'exclusion de taille, et des ions métalliques immobilisés.

2. Dispositif selon la revendication 1, comprenant en outre au moins l'une des caractéristiques suivantes : un filtre poreux (5), un fritté poreux (5) ou une membrane (5), qui est disposé(e) entre la sortie (3) de l'espace creux et la phase stationnaire (4) et qui retient la phase stationnaire (4) dans l'espace creux, au moins un dispositif de fermeture (6) pour fermer l'entrée (2) et/ou la sortie (3) de l'espace creux, au moins un récipient collecteur pour recueillir la phase mobile (éluat) s'écoulant de la sortie (3) après réalisation de l'élution.

3. Dispositif selon l'une quelconque des revendications 1 ou 2, **caractérisé en ce que** les ions métalliques immobilisés sont des cations métalliques divalents, trivalents ou tétravalents ou des mélanges de ceux-ci, de préférence choisis parmi le groupe constitué de Zn²⁺ Co²⁺ Cu²⁺ Ni²⁺, Fe²⁺, Mn²⁺, Al³⁺, Co³⁺, Ga³⁺ et Zr⁴⁺ ou une combinaison de ceux-ci, de manière particulièrement préférée au moins un cation métallique divalent, choisi parmi le groupe constitué de Zn²⁺, Co²⁺, Cu²⁺ et Ni²⁺, facultativement en association avec les ions Al³⁺, en particulier les ions Cu²⁺, facultativement en association avec les ions Al³⁺.

4. Dispositif selon l'une quelconque des revendications 1 à 3, **caractérisé en ce que** les ions métalliques sont immobilisés à l'aide d'au moins un groupe de liaison sur au moins une partie de la phase stationnaire, le groupe de liaison présentant de préférence la formule générale A-R-Z, dans laquelle A représente un groupe d'ancrage pour une fixation covalente du groupe de liaison sur au moins une partie de la phase stationnaire, Z représente un groupe de tête multidentate pour la chélation des ions métalliques et R représente une chaîne hydrocarbonée linéaire, ramifiée ou cyclique, laquelle relie le groupe d'ancrage A au groupe de tête Z et qui peut être substituée ou non et dans laquelle un ou plusieurs atomes de carbone peuvent être remplacés par des hétéroatomes, et étant de préférence choisi parmi le groupe constitué de l'acide iminodiacétique (IDA), l'acide nitrilotriacétique (NTA), l'acide aspartique N-carboxyméthylé (CM-Asp) et l'amine Tris-(2-éthylaminoéthyl) (TREN).

5. Dispositif selon l'une quelconque des revendications 1 à 4, **caractérisé en ce que** la résine poreuse de chromatographie est un silicate à réticulation transversale ou un polymère organique, laquelle forme lorsqu'on la mélange à de l'eau, à une solution aqueuse ou à un tampon aqueux une matrice de gel avec des pores internes de taille définie (résine de chromatographie d'exclusion de taille), de préférence choisie parmi le groupe contenant la dextrane, l'agarose et le polyacrylamide ou des mélanges de ceux-ci.

6. Dispositif selon l'une quelconque des revendications 1 à 5, **caractérisé en ce que** la phase stationnaire (4) comprend en plus de l'au moins une résine poreuse de chromatographie (résine de chromatographie d'exclusion de taille) au moins un autre matériau sur lequel les ions métalliques sont présents de manière immobilisée.

7. Dispositif selon la revendication 6, **caractérisé en ce que**
a) dans le dispositif de chromatographie, la résine poreuse de chromatographie et le matériau supplémentaire
i) sont disposés sensiblement séparés l'un de l'autre, de préférence sous la forme de deux couches successives dans le sens de l'écoulement de la phase mobile de l'entrée (2) vers la sortie (3), de manière particulièrement préférée de sorte que le matériau auquel sont immobilisés les ions métalliques est disposé comme couche supérieure et la résine de chromatographie d'exclusion de taille comme couche inférieure, ou
ii) sont présents en grande partie comme mélange homogène, ou
b) les ions métalliques sont immobilisés eux-mêmes sur la résine de chromatographie d'exclusion de taille.

8. Dispositif selon l'une quelconque des revendications 1 à 7, **caractérisé en ce que** le dispositif de chromatographie
i) comprend exactement un espace creux (2) rempli d'une phase stationnaire, et le dispositif de chromatographie est façonné de préférence sous forme d'une colonne de centrifugation (*spin column*) ou
ii) comprend pour l'isolement parallèle et/ou la purification d'acides nucléiques double brin issus de différents échantillons plusieurs espaces creux remplis respectivement avec la phase stationnaire (4), lesquels sont disposés de manière parallèle, de préférence sous forme de plaque de filtre.

9. Dispositif selon l'une quelconque des revendications 1 à 8, **caractérisé en ce que** le corps de base est constitué d'un matériau contenant un polymère organique, de préférence choisi parmi le groupe constitué de polyéthylène (PE), polypropylène (PP), polycarbonate, polystyrène, polyphénylsulfone ou polysulfone.

10. Utilisation d'un dispositif selon l'une quelconque des revendications 1 à 9 pour l'isolement et/ou la purification chromatographique d'acides nucléiques double brin issus d'un mélange de tels acides nucléiques avec des acides nucléiques simple brin, oligonucléotides, mononucléotides, sels et/ou d'autres impuretés, de préférence pour l'isolement et/ou la purification de produits d'amplification d'une PCR d'un mélange réactionnel.

11. Procédé pour l'isolement et/ou la purification chromatographique d'acides nucléiques double brin issus d'un mélange de tels acides nucléiques avec des acides nucléiques simple brin, oligonucléotides, mononucléotides, sels et/ou d'autres impuretés, de préférence pour l'isolement et/ou la purification de produits d'amplification d'une PCR d'un mélange réactionnel, comprenant les étapes suivantes :
1. la fourniture d'un échantillon contenant au moins des acides nucléiques double brin sous forme d'une solution aqueuse,
2. l'application de cette solution sur le côté de la phase stationnaire orienté vers l'entrée d'un dispositif de chromatographie selon l'une quelconque des revendications 1 à 9,
3. l'élution des acides nucléiques double brin et le recueil simultané de l'éluat,
**caractérisé en ce que** la séparation des acides nucléiques double brin des autres composants contenus dans l'échantillon s'effectue lors d'une étape de chromatographie.

12. Procédé selon la revendication 11, **caractérisé en ce que** les acides nucléiques double brin représentent des acides désoxyribonucléiques (ADN) double brin, de préférence les ADN double brin linéaires obtenus comme produit d'amplification d'une PCR.

13. Procédé selon l'une quelconque des revendications 11 ou 12 **caractérisé en ce que** l'élution des acides nucléiques double brin s'effectue par l'application d'une force de centrifugation sur le dispositif de chromatographie.

14. Procédé selon la revendication 13, comprenant en outre une étape de préparation de la matrice de gel dans l'espace creux du dispositif de chromatographie en mettant en suspension une résine poreuse de chromatographie dans de l'eau, une solution aqueuse ou un tampon aqueux, en laissant gonfler la résine dans la suspension pour former la matrice de gel et en éliminant l'excès d'eau, le solvant aqueux ou le tampon aqueux par centrifugation du dispositif de chromatographie contenant la résine gonflée avant d'appliquer la solution d'échantillon sur la phase stationnaire.

15. Kit pour l'isolement et/ou la purification d'acides nucléiques double brin, de préférence d'ADN double brin, comprenant un dispositif de chromatographie selon l'une quelconque des revendications 1 à 10 ou les produits de départ de celui-ci et le cas échéant une solution tampon pour l'ajustement du pH de la solution d'échantillon avant la purification chromatographique, la solution tampon étant choisie de préférence parmi le groupe contenant Mops, Mes et Hepes et des solutions aqueuses d'acétates ou phosphates de métaux alcalins, de préférence une solution d'acétate de sodium aqueuse.
